(19) Europäisches Patentamt
European Patent Office
Office européen des brevets

(11) Veröffentlichungsnummer: 0 305 332
A2

# (12) EUROPÄISCHE PATENTANMELDUNG

(21) Anmeldenummer: 88810562.4

(22) Anmeldetag: 17.08.88

(51) Int. Cl.$^4$: C 07 D 405/04
C 07 D 409/04,
C 07 D 401/04,
C 07 D 217/24,
C 07 D 221/20,
C 07 D 311/76,
C 07 D 311/96,
C 07 D 335/06, C 07 D 335/04

(30) Priorität: 26.08.87 CH 3262/87

(43) Veröffentlichungstag der Anmeldung:
01.03.89 Patentblatt 89/09

(84) Benannte Vertragsstaaten:
AT BE CH DE ES FR GB GR IT LI LU NL SE

(71) Anmelder: CIBA-GEIGY AG
Klybeckstrasse 141
CH-4002 Basel (CH)

JANSSEN PHARMACEUTICA N.V.
Turnhoutsebaan 30
B-2340 Beerse (BE)

(72) Erfinder: Schneider, Hans-Dieter, Dr.
Efringerstrasse 32
D-7858 Weil am Rhien (DE)

Lutz, William R., Dr.
Auf der Bischoffhöhe 104
CH-4125 Riehen (CH)

Szczepanski, Henry, Dr.
Bodenmatt
CH-4323 Wallbach (CH)

Töpfl, Werner, Dr.
Dorneckstrasse 68
CH-4143 Dornach (CH)

(54) **Imidazol-Derivate.**

(57) Imidazol-Derivate der Formel I

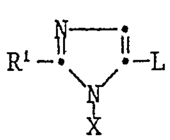

(I),

sowie stereochemisch isomere Formen davon und deren Salze, haben nützliche herbizide Eigenschaften. Die Substituenten $R^1$, L und X haben die hierin definierten Bedeutungen.

EP 0 305 332 A2

**Beschreibung**

**Imidazol-Derivate**

Die vorliegende Erfindung betrifft neue herbizid wirksame Isochroman-, Isothiochroman- und Tetrahydroisochinolin-imidazol-Derivate, diese Wirkstoffe enthaltende agrochemische Mittel und ein Verfahren zur Bekämpfung von Unkräutern, vorzugsweise zur selectiven Bekämpfung von Unkräutern in Nutzpflanzenkulturen. Ferner betrifft die Erfindung auch ein Verfahren zur Herstellung der neuen Verbindungen, sowie neue Zwischenprodukte.

Die herbizid wirksamen Isochroman-, Isothiochroman- und Tetrahydroisochinolin-imidazol-Derivate entsprechen der Formel I

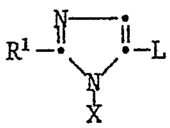

(I),

den stereochemisch isomeren Formen davon, sowie deren Salzen, worin X einen Rest der Formel

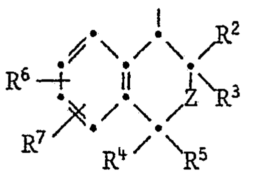

oder

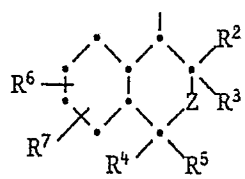

.

$R^1$ Wasserstoff oder -SH, und
L Cyano, -COOH, $-COOR^8$, $-COSR^8$, $-CONR^9R^{10}$, $-CO-R^{11}$, $-CH_2-O-R^{12}$ oder eine Gruppe

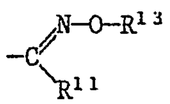

oder

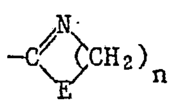

bedeuten, wobei

bedeuten, wobei E für Sauerstoff, Schwefel, $-N(C_1-C_4Alkyl)-$ oder -NH- ,
n für die Zahl zwei oder drei,
Z für Sauerstoff, Schwefel, -SO-, $-SO_2-$ oder $-NR^{14}-$,
$R^2$ und $R^3$ unabhängig voneinander für Wasserstoff, $C_1$-$C_4$-Alkyl, $C_2$-$C_4$-Alkenyl oder $C_2$-$C_4$-Alkinyl oder gemeinsam für eine spirocyclisch verknüpfte $C_2$-$C_6$-Alkylenkette,
$R^4$ und $R^5$ unabhängig voneinander für Wasserstoff oder $C_1$-$C_4$-Alkyl oder gemeinsam für eine Oxofunktion,
$R^6$ für Wasserstoff, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio oder Nitro,
$R^7$ für Wasserstoff, $C_1$-$C_4$-Alkoxy, Halogen oder $C_1$-$C_4$-Alkyl,
$R^8$ für $C_1$-$C_6$-Alkyl, $C_3$-$C_7$-Cycloalkyl, $C_2$-$C_6$-Alkoxyalkyl, Benzyl, $-CR^{15}R^{16}$-$C_2$-$C_4$-Alkenyl oder $-CR^{15}R^{16}$-$C_2$-$C_4$-Alkinyl,
$R^9$ für Wasserstoff, $C_1$-$C_4$-Alkyl, $C_1$-$C_4$-Alkoxy, Phenyl, Benzyl, $-CR^{15}R^{16}$-$C_2$-$C_4$-Alkenyl oder $-CR^{15}R^{16}$-$C_2$-$C_4$-Alkinyl,
$R^{10}$ für Wasserstoff oder $C_1$-$C_4$-Alkyl, oder
$R^9$ und $R^{10}$ zusammen mit dem sie tragenden Stickstoffatom für einen Pyrrolidin-, Piperidin- oder Morpholinrest,
$R^{11}$ für $C_1$-$C_6$-Alkyl, $C_3$-$C_7$-Cycloalkyl, $C_1$-$C_4$-Halogenalkyl oder gegebenenfalls durch $C_1$-$C_4$-Alkyl, $C_1$-$C_4$-Alkoxy, Nitro oder Halogen substituiertes Phenyl,
$R^{12}$ für Wasserstoff, $C_1$-$C_6$-Alkyl, gegebenenfalls durch $C_1$-$C_4$-Alkyl, $C_1$-$C_4$-Alkoxy, Halogen oder Nitro substituiertes Phenyl, $C_1$-$C_6$-Alkylcarbonyl, $C_1$-$C_4$-Halogenalkylcarbonyl oder gegebenenfalls durch $C_1$-$C_4$-Alkyl, $C_1$-$C_4$-Alkoxy, Halogen oder Nitro substituiertes Benzoyl,
$R^{13}$ für $C_1$-$C_4$-Alkyl, $-CR^{15}R^{16}$-$C_2$-$C_4$-Alkenyl, Benzyl oder $-CR^{15}R^{16}$-$C_2$-$C_4$-Chloralkenyl,
$R^{14}$ für Wasserstoff, $C_1$-$C_4$-Alkyl, $C_1$-$C_4$-Alkylcarbonyl, $C_1$-$C_4$-Alkoxycarbonyl, Benzyloxycarbonyl, Benzoyl oder Benzyl,
und
$R^{15}$ und $R^{16}$ unabhängig voneinander für Wasserstoff oder $C_1$-$C_4$-Alkyl stehen.

5-Imidazolcarbonsäure-Derivate mit Tetralinyl- und Indanyl-Substituenten in der Position 1 sind aus der

Europäischen Patentanmeldung EP-A-207 563 mit ihren biologischen Wirkungen sowie Verfahren zu ihrer Herstellung bekannt.

Ueberraschenderweise besitzen die Verbindungen der Formel I starke herbizide Eigenschaften, welche es erlauben, Unkräuter zu bekämpfen. Diese Eigenschaft gewinnt dadurch an Bedeutung, dass einige Nutzpflanzenkulturen nicht durch die Behandlung mit Verbindungen der Formel I geschädigt werden oder, dass nur bei sehr hohen Dosierungen geringfügige Schädigungen der Kultur auftreten. Somit sind die Verbindungen der Formel I wertvolle Selektivherbizide in Nutzpflanzenkulturen wie Getreide, Zuckerrüben, Raps, Sojabohnen, Reis und Mais. Besonders in Reiskulturen kann ein breiter Bereich von Aufwandmengen angewendet werden, insbesondere dann, wenn es sich bei den Reiskulturen um verpflanzten Reis handelt und wenn die Verbindungen der Formel I nach der Verpflanzung ausgebracht werden.

Die aktiven Wirkstoffe der Formel I werden üblicherweise bei Aufwandmengen zwischen 0,01 und 5,0 kg Aktivsubstanz pro Hektar angewendet, um zufriedenstellende Resultate zu erzeugen. Wenn die Umweltbedingungen es erfordern, können die Aufwandmengen in geeigneten Fällen die oben angegebenen Grenzwerte überschreiten. Die bevorzugten Aufwandmengen liegen jedoch im allgemeinen zwischen 0,02 kg und 1,0 kg Wirkstoff pro Hektar.

In den Definitionen ist unter Alkyl geradkettiges oder verzweigtes Alkyl zu verstehen; z.B. Methyl, Aethyl, n-Propyl, i-Propyl oder die vier isomeren Butyl. Unter Alkoxy ist zu verstehen: Methoxy, Aethoxy, Propyl oxy oder die vier isomeren Butyloxy, insbesondere aber Methoxy, Aethoxy oder Isopropyloxy. Alkylthio steht für Methylthio, Aethylthio, Propylthio, Isopropylthio oder Butylthio, insbesondere aber Methylthio.

Halogen bedeutet in den obigen Definitionen Fluor, Chlor, Brom oder Jod, worunter Fluor und Chlor bevorzugt sind. Alkenyl bedeutet beispielsweise Vinyl, Allyl, 1-Methylvinyl, 3-Butenyl, 2-Butenyl, 1-Butenyl, 1-Propenyl, Methallyl oder 3-Methyl-2-butenyl, wobei Vinyl, Allyl und Methallyl bevorzugt sind. Beispiele für Alkinyl sind Aethinyl, 1-Propinyl, Propargyl, 1-Butinyl, 2-Butinyl oder 3-Butinyl, vorzugsweise aber Aethinyl oder Propargyl. Cycloalkyl bedeutet im allgemeinen Cyclopropyl, Cyclobutyl, Cyclopentyl, Cyclohexyl oder Cycloheptyl, vorzugsweise Cyclopentyl oder Cyclohexyl. Reste, welche aus den gegebenen Bedeutungen kombiniert werden können, wie Halogenalkyl, Halogenalkoxy, Alkoxyalkyl, Halogenalkylthio oder Halogenalkylcarbonyl haben die entsprechend kombinierten konkreten Gruppenmitglieder. Wichtige Beispiele dafür sind: Trifluormethyl, Chlormethyl, 2,2,2-Trifluoräthyl, Trifluormethoxy, Difluormethoxy, 2,2,2-Trifluoräthoxy, 2-Chloräthoxy, Methoxymethyl, Methoxyäthyl, Aethoxyäthyl, Difluormethylthio, Chlormethylcarbonyl, Trifluormethylcarbonyl oder Brommethylcarbonyl.

Die Radikale X, welche über ein Stickstoffatom an den Imidazolring gebunden sind, umfassen die folgenden grundlegenden Strukturen, die ihrerseits in unsubstituierter Form oder mit den genannten Substituenten $R^2$, $R^3$, $R^4$, $R^5$, $R^6$, $R^7$ und $R^{14}$ vorliegen können. In den Fällen der Verknüpfung von zwei aliphatischen Ringsystemen sind sowohl cis- als auch trans-Ringverknüpfungen gemeint.

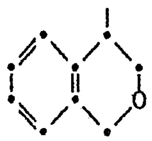 , 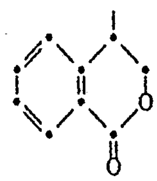 , 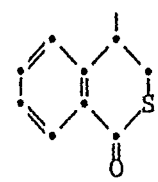 ,

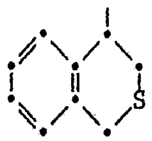 , 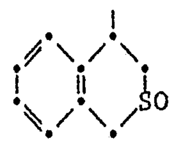 , 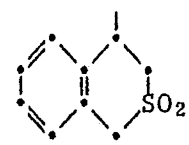 ,

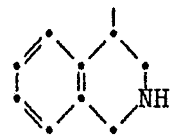 ,

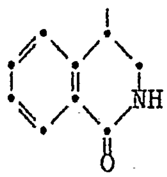 , 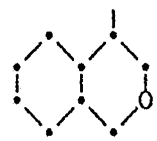 , 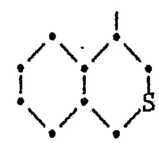 ,

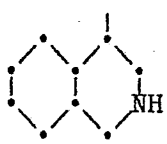 , 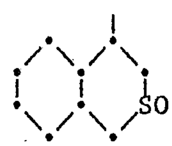 oder 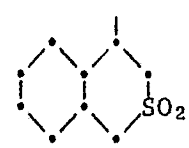 .

Die Reste $R^2$ und $R^3$ sind an das gleiche Kohlenstoffatom gebunden. Somit können sie zusammen mit diesem Kohlenstoffatom einen spirocyclischen Ring bilden. Typische Vertreter solcher spirocyclischen Ringe sind Cyclopropan-, Cyclobutan-, Cyclopentan-, Cyclohexan- und Cycloheptan-Ringe.

Die Erfindung umfasst ebenfalls die Salze, die die Verbindungen der Formel I mit organischen oder anorganischen Basen wie Aminen, Alkalimetallbasen und Erdalkalimetallbasen oder quaternären Ammoniumbasen oder mit organischen oder anorganischen Säuren wie Mineralsäuren, Sulfonsäuren, Carbonsäuren oder phosphorhaltigen Säuren bilden können.

Beispiele salzbildender Mineralsäuren sind Fluorwasserstoffsäure, Chlorwasserstoffsäure, Bromwasserstoffsäure, Jodwasserstoffsäure, Schwefelsäure, Salpetersäure, Chlorsäure, Perchlorsäure oder Phosphorsäure. Bevorzugte salzbildende Sulfonsäuren sind Toluolsulfonsäuren, Benzolsulfonsäure, Methansulfonsäure oder Trifluormethansulfonsäure. Als salzbildende Carbonsäuren seien vorzugsweise Essigsäure, Trifluoressigsäure, Benzoesäure, Chloressigsäure, Phthalsäure, Maleinsäure, Malonsäure und Zitronensäure genannt. Phosphor-haltige Säuren sind die verschiedenen Phosphonsäuren, phosphonigen Säuren und Phosphinsäuren.

Bevorzugte salzbildenden Alkalimetallhydroxide und Erdalkalimetallhydroxide sind die Hydroxide von Lithium, Natrium, Kalium, Magnesium oder Calcium, insbesondere aber die von Natrium oder Kalium. Beispiele von geeigneten salzbildenden Aminen sind primäre, sekundäre und tertiäre aliphatische und aromatische Amine wie Methylamin, Aethylamin, Propylamin, Isopropylamin, die vier Butylaminisomeren, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und Isochinolin. Bevorzugte Amine sind Aethylamin, Propylamin, Diäthylamin ode Triäthylamin, insbesondere aber Isopropylamin, Diäthanolamin und 1,4-Diazabicyclo[2.2.2]octan. Beispiele für quaternäre Ammoniumbasen sind im allgemeinen die Kationen von Halogenammoniumsalzen, beispielsweise das Tetramethylammoniumkation, das Trimethylbenzylammoniumkation, das Tetraäthylammoniumkation, das Trimethyläthylammoniumkation und auch das Ammoniumkation.

Die Erfindung umfasst ebenfalls alle optischen Isomeren der Verbindungen der Formel I. Diese können auftreten, wenn der Rest X in der Position 1 des Imidazolrings und/oder die Gruppe L der Verbindungen der Formel I asymmetrisch substituierte Kohlenstoffatome enthalten. Sofern keine nähere Angabe gemacht wird, umfasst die chemische Bezeichnung von Verbindungen deren Mischungen aller stereochemisch isomeren Formen. Die Mischungen enthalten alle Diastereomeren und Enantiomeren der zugrundeliegenden Molekülstruktur.

Die reinen isomeren Formen dieser optisch aktiven Verbindungen können aus den Mischungen durch

übliche Trennungsmethoden erhalten werden. Wird im Einzelfall eine spezifische stereochemische Form gewünscht, so wird diese Verbindung vorzugsweise durch stereoselektive Syntheseverfahren hergestellt. In diesen Verfahren kommen mit Vorteil reine Formen der optisch aktiven Ausgangsmaterialien zur Anwendung.

Unter den Verbindungen der Formel I sind diejenigen bevorzugt, in denn entweder

a) $R^1$ für Wasserstoff steht, oder

b) L für $-COOR^8$, $-CONR^9R^{10}$ oder $-CO-R^{11}$ steht, oder

c) X für den Rest

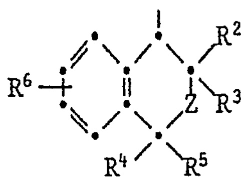

steht, worin

steht, worin $R^2$ und $R^3$ unabhängig voneinander Wasserstoff oder $C_1$-$C_4$-Alkyl, $R^4$ und $R^5$ Wasserstoff oder gemeinsam für eine Oxofunktion, $R^6$ Wasserstoff, $C_1$-$C_4$-Alkyl, $C_1$-$C_4$-Alkoxy oder Halogen und Z Sauerstoff, -NH-, $-NCH_3-$ oder Schwefel bedeuten, oder

d) X für den Rest

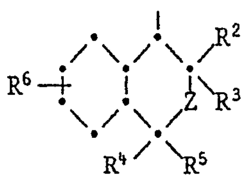

steht, worin

steht, worin die Ringverknüpfung in trans-Stellung vorliegt, $R^2$ und $R^3$ unabhängig voneinander Wasserstoff oder $C_1$-$C_4$-Alkyl, $R^4$ und $R^5$ Wasserstoff, $R^6$ Wasserstoff, $C_1$-$C_4$-Alkyl oder Halogen und Z Sauerstoff, -NH-, $-NCH_3$-oder Schwefel bedeuten.

Unter den Verbindungen der Gruppe b) sind solche Untergruppen bevorzugt, in denen entweder L für $C_1$-$C_4$-Alkoxycarbonyl oder für $C_1$-$C_4$-Alkylcarbonyl oder für $-CO-NR^9R^{10}$ steht, worin $R^9$ und $R^{10}$ unabhängig voneinander Wasserstoff oder $C_1$-$C_4$-Alkyl bedeuten.

Unter den Verbindungen der Gruppe c) sind diejenigen bevorzugt, worin $R^1$ für Wasserstoff und L für $C_1$-$C_4$-Alkoxycarbonyl, $C_1$-$C_4$-Alkylcarbonyl, Carbamoyl, Methylcarbamoyl oder Dimethylcarbamoyl stehen.

Unter den Verbindungen der Gruppe d) sind diejenigen bevorzugt, worin $R^1$ für Wasserstoff und L für $C_1$-$C_4$-Alkoxycarbonyl, $C_1$-$C_4$-Alkylcarbonyl, Carbamoyl, Methylcarbamoyl oder Dimethylcarbamoyl stehen.

Als bevorzugte Einzelverbindungen der vorliegenden Erfindung sind zu nennen:

1-(3-Methyl-isochroman-4-yl)-5-imidazolcarbonsäure-methylester,
1-(3,3-Dimethyl-isochroman-4-yl)-5-imidazolcarbonsäure-methylester,
1-(3-Methyl-isothiochroman-4-yl)-5-imidazolcarbonsäure-methylester,
1-(3,3-Dimethyl-isothiochroman-4-yl)-5-imidazolcarbonsäure-methylester,
1-(3-Methyl-1,2,3,4-tetrahydroisochinolin-4-yl)-5-imidazolcarbonsäure-methylester,
1-(2,3-Dimethyl-1,2,3,4-tetrahydroisochinolin-4-yl)-5-imidazolcarbonsäure-methylester,
1-(3,3-Dimethyl-1,2,3,4-tetrahydroisochinolin-4-yl)-4-imidazolcarbonsäure-methylester und
1-(3,3-Dimethyl-1-oxo-isochroman-4-yl)-5-imidazolcarbonsäure-methylester.

Die erfindungsgemässen Verbindungen der Formel I werden im allgemeinen nach den folgenden Methoden hergestellt.

Nach einem ersten Verfahren erhält man Verbindungen der Formel I, worin $R^1$ für Wasserstoff steht, indem man ein Amin der Formel II

$X-NH_2$ (II)

worin X die unter Formel I gegebene Bedeutung hat, mit einem N-Cyan-Imidoameisensäureester der Formel III

$R-O-CH=N-CN$ (III)

worin R für $C_1$-$C_4$-Alkyl steht, kondensiert, das entstehende n-Cyanoformamidin der Formel IV

$X-NH-CH=N-CN$ (IV)

worin X die unter Formel I gegebene Bedeutung hat, mit einem Halogenessigsäureester der Formel V

Hal-$CH_2$-L (V)

worin Hal für Chlor oder Brom und L für $C_1$-$C_6$-Alkoxycarbonyl stehen, in Gegenwart einer Base alkyliert, das entstehende Zwischenprodukt der Formel VI

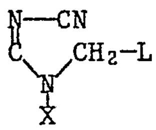

(VI)

worin X die unter Formel I gegebene Bedeutung hat und L für $C_1$-$C_6$-Alkoxycarbonyl steht, in Gegenwart einer Base zum 4-Amino-imidazol der Formel VII

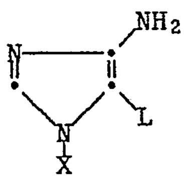

(VII)

worin X die unter Formel I gegebene Bedeutung hat und L für $C_1$-$C_6$-Alkoxycarbonyl steht, cyclisiert und diese Verbindung unter Diazotierung und Stickstoffabspaltung in das Produkt der Formel I, worin L für $C_1$-$C_6$-Alkoxycarbonyl und $R^1$ für Wasserstoff stehen, überführt und gewünschtenfalls die Gruppe L in 5-Stellung gemäss den übrigen Bedeutungen der Definition unter Formel I derivatisiert.

Der besondere Vorteil dieses Verfahrens ist in der Möglichkeit zu sehen, das Verfahren in einem einzigen Reaktionsgefäss - ohne Isolierung der Zwischenprodukte - durchführen zu können. Ferner können die ersten beiden Reaktionsschritte zur Alkylierung des primären Amins der Formel II mit den Produkten der Formeln III und V beliebig miteinander vertauscht werden.

Nach zweitem Verfahren erhält man die Verbindungen der Formel I, indem man das Amin der Formel II

X-$NH_2$ (II)

worin X die unter Formel I gegebene Bedeutung hat, mit einem Halogenessigsäureester der Formel V

Hal-$CH_2$-L (V)

worin Hal für Chlor oder Brom und L für $C_1$-$C_6$-Alkoxycarbonyl stehen, in Gegenwart einer Base alkyliert, den entstehenden Glycinester der Formel VIII

X-NH-$CH_2$-L (VIII)

worin X die unter Formel I gegebene Bedeutung hat und L für $C_1$-$C_6$-Alkoxycarbonyl steht, in Gegenwart von Essigsäureanhydrid mit Ameisensäure formyliert, das entstehende Zwischenprodukt der Formel IX

HCO- -$CH_2$-L (IX)

worin X die unter Formel I gegebene Bedeutung hat und L für $C_1$-$C_6$-Alkoxycarbonyl steht, in Gegenwart einer Alkalibase mit einem Ameisensäure-$C_1$-$C_4$-alkylester umsetzt, das entstehende Zwischenprodukt der Formel X

HCO- — =CH-O-M (X)

worin X die unter Formel I gegebene Bedeutung hat und L für $C_1$-$C_6$-Alkoxycarbonyl und M für ein Alkalikation steht, mit Thiocyansäure zum Produkt der Formel I, worin $R^1$ für -SH und L für $C_1$-$C_6$-Alkoxycarbonyl stehen, cyclisiert und diese Verbindung gewünschtenfalls in der 2-Stellung desulfuriert und gewünschtenfalls die Gruppe L in 5-Stellung gemäss den übrigen Bedeutungen der Definition unter Formel I derivatisiert.

Bei der Derivatisierung des $C_1$-$C_6$-Alkoxycarbonylrestes in 5-Stellung wird in bekannter Weise modifiziert, beispielsweise durch Verseifung, Veresterung, Umesterung, Amidierung, Umamidierung, Oximierung oder Reduktion. Gewünschtenfalls kann auch eine Salzbildung am basischen Stickstoffatom des Imidazolringes

erfolgen.

Die Cyanoverbindung (L = -CN) erhält man, indem man das Carbonsäureamid (L = $-CONH_2$) dehydratisiert. Als Mittel hierzu kommen beispielsweise Phosphorpentachlorid, Phosphoroxychlorid, Thienylchlorid, Phosphorpentoxid und Acetanhydrid in Frage. Die Reaktionstemperatur hängt von der Wahl des Dehydratisierungsmittels ab und liegt im allgemeinen zwischen +20°C und +120°C. Gegebenenfalls können auch inerte organische Lösungsmittel zugesetzt werden. Die Ester, Thiolester oder Amidderivate (L = $-COOR^8$, $-COSR^8$, $-CONR^9R^{10}$) lassen sich aus den Carbonsäuren (L = -COOH) oder den daraus erhältlichen aktivierten Verbindungen wie Säurehalogeniden oder Anhydriden oder gemischten Anhydriden durch Umsetzung mit den entsprechenden Alkoholen, Thiolen oder Aminverbindungen herstellen. Ester und Thiolester lassen sich ausserdem auch durch Alkylierung der Carbonsäuren mit Halogenalkylverbindungen in Gegenwart von Basen erhalten. Die heterocyclischen Verbindungen der Formel I mit

L = 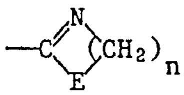

sind durch Umsetzung der entsprechenden Carbonsäuren, bzw. deren aktivierten Vertreter, wie ihren Säurehalogeniden, mit bifunktionellen Verbindungen wie Diaminen, Aminoalkohlen oder Aminothiolen nach an sich bekannten Verfahren zugänglich. Die Keton-Derivate (L = $-CO-R^{11}$) können aus den Estern durch Reduktion zur Aldehydstufe, Alkylierung zum sekundären Alkohol mittels Grignard-Addition und anschliessende Oxidation erhalten werden. Die Aether-Derivate (L = $-CH_2-O-R^{12}$) können aus den Estern durch Reduktion zum Alkohol und gewünschtenfalls angeschlossene Verätherung erhalten werden.

Die Isothiochroman-2-oxide und Isothiochroman-2,2-dioxide der Formel I können durch Behandlung der entsprechenden Isothiochromane oder deren Salze der Formel I mit Oxidationsmitteln wie zum Beispiel Wasserstoffperoxid, m-Chlorperbenzoesäure oder Natrium-meta-periodat hergestellt werden.

Die Verbindungen der Formel I, worin $R^4$ und $R^5$ zusammen für eine Oxofunktion stehen, können dann durch Oxidation aus den entsprechenden Verbindungen erhalten werden, worin $R^4$ und $R^5$ für Wasserstoff stehen, wenn $R^4$ und $R^5$ an ein benzylisches Kohlenstoffatom gebunden ist, das heisst, wenn der $R^6$ und $R^7$ tragende Ring aromatisch ist. Ist der letztgenannte Ring gesättigt, kann die Synthese der Verbindungen, worin $R^4$ und $R^5$ eine Oxofunktion bilden, beispielsweise nach dem folgenden Syntheseschema erfolgen:

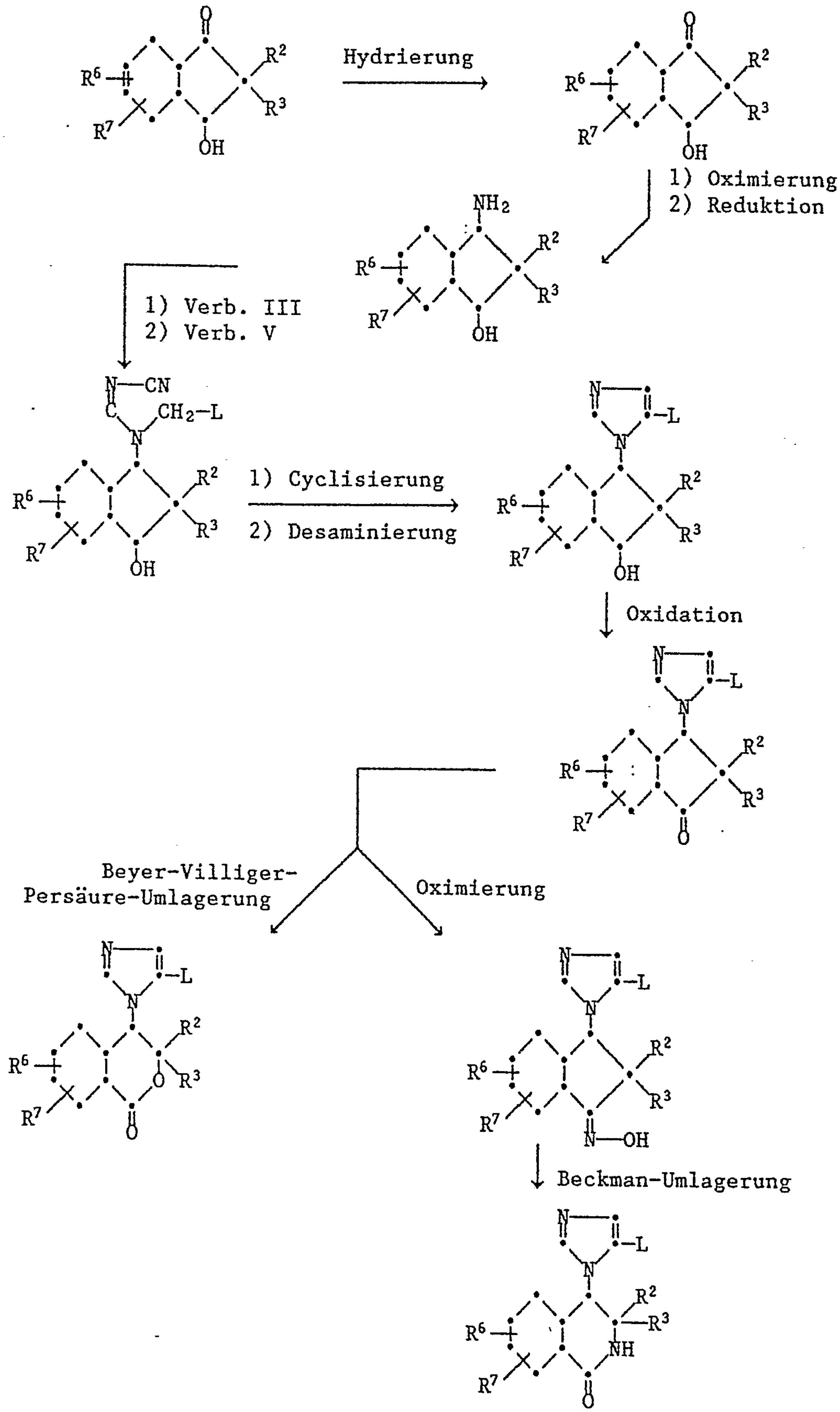
Hydrierung
1) Oximierung
2) Reduktion
1) Verb. III
2) Verb. V
1) Cyclisierung
2) Desaminierung
Oxidation
Beyer-Villiger-
Persäure-Umlagerung
Oximierung
Beckman-Umlagerung

Die einzelnen Reaktionsschritte dieses Schemas sind in der Literatur in analoger Weise beschrieben. Die Reaktionsbedingungen entsprechend den dabei üblichen Konditionen.

Die Zwischenprodukte der Formel VII sind neu. Sie werden speziell für die Synthese der Verbindungen der Formel I entwickelt. Sie sind daher Gegenstand der vorliegenden Erfindung. Die Amine der Formel II sind zum grossen Teil neu. Diese neuen Verbindungen der Formel II bilden ebenfalls einen Erfindungsgegenstand. Sie entsprechen der Formel II unter Ausnahme der Verbindungen 4-Amino-2-methyl-1,2,3,4tetrahydroisochinolin, 4-Amino-2-äthyl-1,2,3,4-tetrahydroisochinolin, 4-Amino-2-benzyl-1,2,3,4-tetrahydroisochinolin und 4-Amino-7-methoxy-1,2,3,4-tetrahydroisochinolin.

Die einzelnen Reaktionsstufen der verschiedenen Verfahren können mit Vorteil unter den anschliessend geschilderten Bedingungen durchgeführt werden.

Die Kondensation (II + III → IV) wird mit Vorteil in einem polaren Lösungsmittel wie Dimethylformamid oder Dimethylacetamid oder in einem Alkohol, dessen Alkylrest der Alkylgruppe des N-Cyan-imidoameisensäureesters der Formel III entspricht, bei einer Temperatur zwischen +20°C und +80°C durchgeführt. Im allgemeinen erfolgt die Reaktion spontan und ist exotherm.

Die Alkylierung des Produkts der Formel IV (IV + V → VI) erfolgt in Gegenwart einer Base als säurebindendes Mittel. Solche Reagenzien können sowohl anorganische als auch organische Basen sein. Beispiele sind Alkalihydroxide, Alkalihydrogencarbonate, Alkalicarbonate, Erdalkalioxide, Erdalkalicarbonate, Alkalialkoholate oder tertiäre Amine, wie Natriumhydroxid, Kaliumhydroxid, Natriumcarbonat, Kaliumhydrogencarbonat, Magnesiumoxid, Calciumoxid, Natriummethylat, Natriumäthylat, Kaliummethylat, Kaliumäthylat, Kalium-tert.butylat, Pyridin, Triäthylamin etc. Die Reaktionstemperaturen liegen im allgemeinen zwischen 0°C und +80°C, vorzugsweise zwischen +10°C und +50°C. Sofern die Reaktion nicht lösungs mittelfrei durchgeführt werden soll, eignen sich insbesondere polare aprotische Lösungsmittel für die Alkylierung wie Dimethylformamid, Dimethylacetamid oder Dimethylsulfoxid.

Die Cyclisierung des Zwischenprodukts der Formel VI zu den 4-Aminoimidazolen der Formel VII findet vorzugsweise unter Katalyse einer Base bei erhöhten Temperaturen statt. Geeignete Bedingungen liegen dann vor, wenn man in einem Alkohol, der dem zur Veresterung der Alkoxycarbonylgruppe verwendeten entspricht, in Gegenwart von katalytischen Mengen des durch Lösen eines Alkalimetalls erzeugten Alkoholats zum Sieden erhitzt. Andererseits lässt sich die Reaktion auch durch Erhitzen der Verbindung der Formel VI mit dem entsprechenden Alkoholat in einem polaren Lösungsmittel wie Dimethylformamid oder Dimethylsulfoxid herbeiführen. Die Reaktionstemperaturen liegen in der Regel zwischen +60°C und +140°C.

Die Desaminierung der 4-Stellung des Imidazolringes (VII → I) erfolgt zweckmässigerweise, indem man das 4-Amino-imidazol in Tetrahydrofuran, Dioxan oder Dimethylformamid mit tert.-Butylnitrit behandelt. Die Diazotierung der Aminogruppe und anschliessende Stickstoffabspaltung kann aber auch in wässrigem saurem Medium mit Natriumnitrit in Gegenwart von Salpetersäure, Salzsäure oder Schwefelsäure und angeschlossenem Verkochen mit hypophosphoriger Säure $H_3PO_2$ erfolgen.

Die Darstellung des Glycinesters der Formel VIII (II + V → VIII) wird unter den gleichen Reaktionsbedingungen durchgeführt wie die oben erläuterte Verfahrensschritt (IV + V → VI).

Die Formylierung des Glycinesters der Formel VIII unter Einsatz von Ameisensäure (VIII → IX) in Gegenwart eines wasserentziehenden Mittels wie Acetanhydrid erfolgt nach an sich für die Darstellung von Carbonsäureamiden üblichen Kondensationsbedingungen. Alternativ kann man die Formylierung auch durch Reaktion mit Ameisensäure in einer Wasserabscheidungsapparatur durch azeotrope Entfernung des Reaktionswassers erreichen.

Die Kondensation der Zwischenprodukte der Formel IX mit einem Ameisensäurealkylester in Gegenwart einer Alkalibase wird üblicherweise in einem inerten Lösungsmittel durchgeführt. Als Alkalibase haben sich Alkalialkoholate oder Alkalihydride erwiesen. Beispiele sind Natriummethylat, Kaliummethylat, Kaliumäthylat, Natriumäthylat, Natriumhydrid oder Lithiumhydrid. Inerte Lösungsmittel sind Alkohole wie Methanol, Aethanol oder Isopropanol oder Aether wie Diäthyläther, Tetrahydrofuran oder Dioxan. Die Reaktionstemperaturen liegen meistens zwischen -20°C und +60°C, vorzugsweise zwischen +10°C und +40°C.

Das Cyclisierungsverfahren der Enaminverbindung der Formel X zu den Produkten der Formel I wird zweckmässigerweise in einem wässrigen inerten Reaktionssystem, beispielsweise in Wasser oder in einer Mischung von Wasser mit Tetrahydrofuran oder Dioxan, bei Temperaturen zwischen +50°C und dem Siedepunkt des Lösungsmittels durchgeführt.

Die Desulfurierung der Produkte der Formel I, worin $R^1$ für -SH steht, kann beispielsweise durch Behandlung mit 15%iger salpetriger Säure bei Temperaturen zwischen +20°C und +40°C erfolgen oder durch Behandlung mit Nickel bei Temperaturen zwischen +50°C und +100°C.

Wird die Synthese von stereochemisch reinen Isomeren beabsichtigt, ist es empfehlenswert, stereoselektive Reaktionsschritte und -bedingungen zu wählen. Andererseits können reine Isomeren häufig durch übliche Trennungsmethoden aus dem Isomerengemisch abgetrennt werden.

Die Ausgangsmaterialien der Formeln III und V sind bekannt, bzw. können analog zu bekannten Verbindungen hergestellt werden.

Die Herstellung der Amine der Formel II erfolgt durch Methoden, die in der Literatur bekannt sind, wie beispielsweise Reduktion von Iminen, Oximen oder Oximderivaten auf katalytische oder elektrochemische Art und Weise oder mit Metallhydriden, wie Lithiumaluminiumhydrid oder Natriumborhydrid/Titantetrachlorid. Eine weitere Möglichkeit ist die reduktive Aminierung von Ketonen auf katalytischem Wege oder mit Hydriden wie Natriumcyanoborhydrid. Die Amine der Formel II kann man auch gewinnen ausgehend von den Ketonen durch

Reaktion mit Ammoniumformiat und anschliessender Hydrolyse der N-Formyl-Derivate. Diese Reaktion ist in der Literatur als Leuckart-Wallach'sche Amin-Darstellung bekannt.

Die zur Herstellung der Amine der Formel II erforderlichen Oxime erfolgt durch an sich bekannte Umsetzung von entsprechenden Ketonen mit Hydroxylamin. Die Oxime der Formel XI ud XII

(XI) 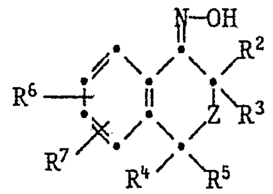

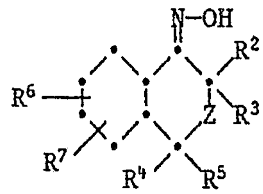 (XII)

worin Z, $R^2$, $R^3$, $R^4$, $R^5$, $R^6$ und $R^7$ die unter Formel I gegebenen Bedeutungen haben, sind neu mit Ausnahme der Verbindungen der Formel XI, worin $R^2$ und $R^3$ gleichzeitig Wasserstoff bedeuten.

Die den Oximen der Formeln XI und XII zugrunde liegenden Ketone sind teilweise bekannt. Ebenso ist die Herstellung der Ketone teilweise in der Literatur beschrieben, wie zum Beispiel Isothiochromanon (J.Amer.Chem.Soc. 85, 2278 (1963)), substituierte Isothiochromanone (Bull.Soc.Chim. France 2225 (1961)), 2-Isochroman-4-on (J.Org.Chem. 50, 2128 (1985)), 4-Keto-1,2,3,4-tetrahydroisochinolin (Advan.Heterocyclic Chem. 15, 99-136 (1973)) und 3-Thiadecal-1-on (Recl.Trav.Chim. Pays-Bas 92, 937 (1973)) oder erfolgt in analoger Weise.

Die Verbindungen der Formel I sind stabil und erfordern keine besonderen Vorichtsmassnahmen für ihre Handhabung.

Bei Anwendung der Verbindung der Formel I in dem angegebenen Bereich der Aufwandmengen zeichnen sich diese Wirkstoffe durch gute selektiv-herbizide Eigenschaften aus, die sie ausgezeichnet zum Einsatz in Kulturen von Nutzpflanzen insbesondere Zuckerrüben, Sojabohnen, Getreide und Mais und ganz besonders Reis befähigen. Teilweise werden dabei auch Unkräuter geschädigt, welchen bisher nur mit Totalherbiziden beizukommen war.

Werden Aufwandmengen angewendet, die den empfohlenen Bereich weit übersteigen, so werden alle behandelten Pflanzen so stark in ihrer Entwicklung geschädigt, dass sie absterben.

Die Erfindung betrifft auch herbizide Mittel, welche die neuen Verbindungen der Formel I enthalten. Ebenso erstreckt sich die Erfindung auf Verfahren zur Bekämpfung von Unkräutern durch die Anwendung dieser neuen Wirkstoffe.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fett säuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niederige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:
"Mc Cutcheon's Detergents and Emulsifiers Annual" MC Publishing Corp., Ridgewood, New Jersey, 1981;
H. Stache, "Tensid-Taschenbuch", 2. Aufl., C. Hanser Verlag, München, Wien, 1981;
M. and J. Ash. "Encyclopedia of Surfactants", Vol. I-III, Chemical Publishing Co., New York, 1980-1981.

Die agrochemischen Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

Emulgierbare Konzentrate:
Aktiver Wirkstoff: 1 bis 20 %, bevorzugt 5 bis 10 %
oberflächenaktive Mittel: 5 bis 30 %, vorzugsweise 10 bis 20 %
flüssiges Trägermittel: 50 bis 94 %, vorzugsweise 70 bis 85 %

Stäube:
Aktiver Wirkstoff: 0,1 bis 10 %, vorzugsweise 0,1 bis 1 %
festes Trägermittel: 99,9 bis 90 %, vorzugsweise 99,9 bis 99 %

Suspensions-Konzentrate:
Aktiver Wirkstoffe: 5 bis 75 %, vorzugsweise 10 bis 50 %
Wasser: 94 bis 25 %, vorzugsweise 88 bis 30 %
oberflächenaktives Mittel: 1 bis 40 %, vorzugsweise 2 bis 30 %

Benetzbares Pulver:
Aktiver Wirkstoff: 0,5 bis 90 %, vorzugsweise 1 bis 80 %
oberflächenaktives Mittel: 0,5 bis 20 %, vorzugsweise 1 bis 15 %
festes Trägermittel: 5 bis 99 %, vorzugsweise 15 bis 90 %

Granulate:
Aktiver Wirkstoff: 0,5 bis 30 %, vorzugsweise 3 bis 15 %
festes Trägermittel: 99,5 bis 70 %, vorzugsweise 97 bis 85 %.

Während als Handelsware eher konzentrierte Mittel bevorzugt werden, verwendet der Endverbraucher in der Regel verdünnte Mittel, Die Anwendungsformen können bis hinab zu 0,001 % an Wirkstoff verdünnt werden.

Die nachfolgenden Beispiele illustrieren die vorliegende Verbindung, ohne eine Begrenzung hiervon darzustellen. Die Herstellungsbeispiele zeigen, wie man die neuen Verbindungen der Formel I erhalten kann. Die biologischen Beispiele und die Formulierungsbeispiele demonstrieren die Anwendung der Wirkstoffe für agrochemische Zwecke.

Herstellungsbeispiele:

Beispiel H1: 1-(3,3-Dimethyl-isothiochroman-4-yl)-5-imidazolcarbonsäure-methylester. (Verbindung 1.016)

a) 3,3-Dimethyl-isothiochroman-4-onoxim.

Ein Gemisch aus 10,0 g 3,3-Dimethyl-isothiochroman-4-on, 8,0 g Hydroxylaminhydrochlorid, 18 ml Pyridin und 60 ml Aethanol wird für 60 Stunden zum Rückfluss erhitzt. Die abgekühlte Reaktionsmischung wird eingedampft, mit Toluol versetzt und nochmals eingedampft. Der Rückstand wird in Dichlormethan aufgekocht, abgekühlt und filtriert. Das Filtrat wird eingedampft und der Rückstand mit Kieselgel chromatographiert (Elutionsmittel: Aethylacetat/Hexan 1:12). Nach Eindampfen des Eluats erhält man 6,0 g 3,3-Dimethyl-isothiochroman-4-onoxim als gelbe Kristalle mit einem Schmelzpunkt von 83-84°C.

b) 4-Amino-3,3-dimethylisothiochroman. (Verbindung 7.04)

Unter einer Stickstoffatmosphäre werden 25,3 g Natriumborhydrid in 435 ml 1,2-Dimethoxyaethan vorgelegt. Bei einer Temperatur von +15 bis +18°C lässt man 36,9 ml Titantetrachlorid zutropfen. Nachdem das Gemisch für 3 Stunden bei einer Temperatur unter +20°C gerührt worden ist, lässt man bei +14 bis +16°C eine Lösung von 33,0 g 3,3-Dimethylisothiochroman-4-onoxim in 135 ml 1,2-Dimethoxyaethan zutropfen. Nach Rühren für 16 Stunden bei Raumtemperatur wird das Gemisch auf Eiswasser gegossen und mit 30 %iger wässriger Ammoniaklösung basisch gestellt. Danach wird zweimal mit Aether extrahiert. Die Aether-Phasen werden mit gesättigter Kochsalzlösung gewaschen, über Natriumsulfat getrocknet und eingedampft. Nach Destillation des Rückstands erhält man 16,7 g 4-Amino-3,3-dimethylisothiochroman als farblose Flüssigkeit von Siedepunkt +80°C bei 0,06 mbar.

c) N-Cyano-N′-(3,3-dimethyl-isothiochroman-4-yl)-formamidin.

16,7 g 4-Amino-3,3-dimethylisothiochroman werden in 10 ml getrocknetem Aethanol vorgelegt. 10,0 g N-Cyan-imidoameisensäure-äthylester werden tropfenweise zugesetzt. Die exotherm verlaufende Reaktion ist nach 2 Stunden beendet. Das Gemisch wird auf 0°C gekühlt, der Niederschlag wird abfiltriert und mit wenig Aethanol gewaschen. Nach dem Trocknen erhält man 14,3 g N-Cyano-N′-(3,3-dimethyl-isothiochroman-4-yl)-formamidin in Form farbloser Kristalle mit einem Schmelzpunkt von 164-166°C.

d) N-Cyano-N′-(3,3-dimethyl-isothiochroman-4-yl)-N′-methoxycarbonylmethyl-formamidin

Einer Mischung aus 23,9 g N-Cyano-N′-(3,3-dimethyl-isothiochroman-4-yl)-formamidin und 18 ml Dimethylsulfoxid setzt man 12,4 g Kalium-tert.-butylat zu. Nach einer Reaktionsdauer von 2,5 Stunden lässt man unter Eiswasserkühlung 10,9 ml Bromessigsäuremethylester zutropfen. Nachdem 16 Stunden bei Raumtemperatur gerührt worden ist, wird das Reaktionsgemisch mit Eiswasser versetzt. Extraktion mit Aether, Waschen der organischen Phase mit gesättigter Kochsalzlösung und Eindampfen der organischen Phase ergibt einen Rückstand, der mit Aether versetzt wird. Der ausgefallene Niederschlag wird abgetrennt und getrocknet. Man erhält 23,9 g N-Cyano-N′-(3,3-dimethyl-isothiochroman-4-yl)-N′-methoxycarbonylmethyl-formamidin mit einem Schmelzpunkt von 141-143°C.

e) 4-Amino-1-(3,3-dimethyl-isothiochroman-4-yl)-5-imidazolcarbonsäuremethylester (Verbindung 9.08)

23,0 g N-Cyano-N′-(3,3-dimethyl-isothiochroman-4-yl)-N′-methoxycarbonylmethyl-formamidin werden in 35 ml Methanol vorgelegt und nach Zusatz von 2,6 ml 30 %iger methanolischer Natriummethylat-Lösung für 16 Stunden zum Sieden erhitzt. Nach Abkühlen wird die Reaktionslösung auf ein Eiswasser/Dichlormethan-Gemisch gegossen. Die organische Phase wird abgetrennt, mit Wasser extrahiert und über Natriumsulfat getrocknet. Nach Filtrieren und Abdampfen erhält man ein braunes Oel, das mit Aether aufgekocht wird. Nach Abkühlen und Anreiben werden 18,3 g 4-Amino-1-(3,3-dimethyl-isothiochroman-4-yl)5-imidazolcarbonsäure-

methylester in Form beiger Kristalle erhalten.

f) Zu einer Lösung von 3,1 ml tert.Butylnitrit in 10 ml Tetrahydrofuran lässt man eine Lösung von 5,0 g 4-Amino-1-(3,3-dimethyl-isothiochroman-4-yl)-5-imidazolcarbonsäure-methylester in 10 ml Tetrahydrofuran zutropfen. Nachdem 16 Stunden bei Raumtemperatur gerührt worden ist, wird das Reaktionsgemisch auf Eiswasser gegossen. Nach Extraktion mit Aether wird die organische Phase mit gesättigter Kochsalzlösung gewaschen, über Natriumsulfat getrocknet, filtriert unf eingedampft. Der Rückstand wird an Kieselgel chromatographisch gereinigt. (Elutionsmittel: Aethylacetat/Hexan 2:5). Man erhält so 3,2 g 1-(3,3-Dimethyl-isothiochroman-4-yl)-5-imidazolcarbonsäure-methylester in Form eines gelben Harzes, das mit Hexan zur Kristallisation gebracht wird. Die kristalline Verbindung hat einen Schmelzpunkt von 108-109° C.

In analoger Weise erhält man die in den Tabellen 1 bis 10 aufgelisteten Verbindungen der Formel I, sowie deren Zwischenprodukte.

Tabelle 1:

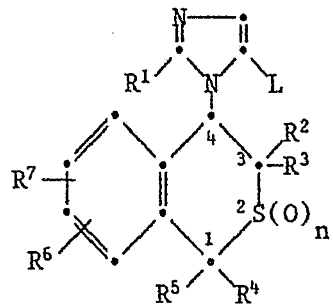

| Verb. Nr. | L | $R^1$ | $R^2$ | $R^3$ | n | $R^4$ | $R^5$ | $R^6$ | $R^7$ | phys. Daten |
|---|---|---|---|---|---|---|---|---|---|---|
| 1.001 | $CO_2CH_3$ | H | H | H | 0 | H | H | H | H | |
| 1.002 | $CO_2CH_3$ | SH | H | H | 0 | H | H | H | H | |
| 1.003 | $CO_2CH_3$ | H | $CH_3$ | H | 0 | H | H | H | H | |
| 1.004 | $CO_2CH_3$ | H | $CH_3$ | H | 0 | H | H | H | H | 3,4-cis, Smp.124-125°C |
| 1.005 | $CO_2CH_3$ | H | $CH_3$ | H | 0 | H | H | H | H | 3,4-trans |
| 1.006 | $CO_2CH_3$ | H | $CH_3$ | H | 0 | =O | | H | H | |
| 1.007 | $CO_2CH_3$ | H | $CH_3$ | H | 0 | $CH_3$ | H | H | H | |
| 1.008 | $CO_2CH_3$ | H | $CH_3$ | H | 0 | $CH_3$ | $CH_3$ | H | H | |
| 1.009 | $CO_2CH_3$ | H | $CH_3$ | H | 0 | $CH_3$ | H | 5-$CH_3$ | H | |
| 1.010 | $CO_2CH_3$ | H | $CH_3$ | H | 0 | $CH_3$ | H | 5-F | H | |
| 1.011 | $CO_2CH_3$ | SH | $CH_3$ | H | 0 | $CH_3$ | H | H | H | |
| 1.012 | $CO_2CH_3$ | H | $CH_3$ | H | 0 | $CH_3$ | H | 6-$CH_3$ | H | |
| 1.013 | $CO_2CH_3$ | H | $CH_3$ | H | 0 | $CH_3$ | H | 6-$OCH_3$ | H | |
| 1.014 | $CO_2CH_3$ | H | $CH_3$ | H | 0 | $CH_3$ | H | 5-$CH_3$ | 8-$CH_3$ | |
| 1.015 | $CO_2CH_3$ | H | $CH_3$ | H | 0 | $C_2H_5$ | H | H | H | |
| 1.016 | $CO_2CH_3$ | H | $CH_3$ | $CH_3$ | 0 | H | H | H | H | Smp.108-109°C |
| 1.017 | $CO_2CH_3$ | SH | $CH_3$ | $CH_3$ | 0 | H | H | H | H | |
| 1.018 | $CO_2CH_3$ | H | $CH_3$ | $CH_3$ | 0 | $CH_3$ | H | H | H | |
| 1.019 | $CO_2CH_3$ | H | $CH_3$ | $CH_3$ | 0 | $CH_3$ | H | H | H | 1,4-cis |
| 1.020 | $CO_2CH_3$ | H | $CH_3$ | $CH_3$ | 0 | $CH_3$ | H | H | H | 1,4-trans |
| 1.021 | $CO_2CH_3$ | H | $CH_3$ | $CH_3$ | 0 | $CH_3$ | $CH_3$ | H | H | |
| 1.022 | $CO_2CH_3$ | H | $CH_3$ | $CH_3$ | 0 | $CH_3$ | H | 6-$CH_3$ | H | |
| 1.023 | $CO_2CH_3$ | H | $CH_3$ | $CH_3$ | 0 | H | H | 6-$CH_3$ | H | |
| 1.024 | $CO_2CH_3$ | H | $CH_3$ | $CH_3$ | 0 | H | H | 5-$CH_3$ | 8-$CH_3$ | |
| 1.025 | $CO_2CH_3$ | H | $CH_3$ | $CH_3$ | 0 | H | H | 7-$OCH_3$ | H | |
| 1.026 | $CO_2CH_3$ | H | $CH_3$ | $CH_3$ | 0 | H | H | 6-$OCH_3$ | H | |
| 1.027 | $CO_2CH_3$ | H | $CH_3$ | $CH_3$ | 0 | H | H | 6-F | H | |
| 1.028 | $CO_2CH_3$ | H | $CH_3$ | $CH_3$ | 0 | H | H | 6-Cl | H | |
| 1.029 | $CO_2CH_3$ | H | $CH_3$ | $CH_3$ | 0 | H | H | 6-Br | H | |
| 1.030 | $CO_2CH_3$ | H | $CH_3$ | $CH_3$ | 0 | H | H | 6-$NO_2$ | H | |
| 1.031 | $CO_2CH_3$ | H | $CH_3$ | $CH_3$ | 0 | H | H | 5-F | H | |
| 1.032 | $CO_2CH_3$ | H | $CH_3$ | $CH_3$ | 0 | H | H | 5-$CH_3$ | H | |
| 1.033 | $CO_2CH_3$ | H | $CH_3$ | $CH_3$ | 0 | H | H | 5-$OCH_3$ | H | |
| 1.034 | $CO_2CH_3$ | H | $CH_3$ | $CH_3$ | 0 | =O | | H | H | |
| 1.035 | $CO_2CH_3$ | H | $-CH_2-CH_2-$ | | 0 | H | H | H | H | |
| 1.036 | $CO_2CH_3$ | SH | $-CH_2-CH_2-$ | | 0 | H | H | H | H | |
| 1.037 | $CO_2CH_3$ | H | $-CH_2-CH_2-$ | | 0 | $CH_3$ | H | H | H | |
| 1.038 | $CO_2CH_3$ | H | H | H | 0 | $CH_3$ | H | H | H | |

Fortsetzung Tabelle 1:

| Verb. Nr. | L | $R^1$ | $R^2$ | $R^3$ | n | $R^4$ | $R^5$ | $R^6$ | $R^7$ | phys. Daten |
|---|---|---|---|---|---|---|---|---|---|---|
| 1.039 | $CO_2CH_3$ | H | H | H | 0 | $CH_3$ | H | H | H | 1,4-cis |
| 1.040 | $CO_2CH_3$ | H | H | H | 0 | $CH_3$ | H | H | H | 1,4-trans |
| 1.041 | $CO_2CH_3$ | H | H | H | 0 | $CH_3$ | $CH_3$ | H | H | |
| 1.042 | $CO_2CH_3$ | SH | H | H | 0 | $CH_3$ | $CH_3$ | H | H | |
| 1.043 | $CO_2CH_3$ | H | H | H | 0 | $C_2H_5$ | H | H | H | |
| 1.044 | $CO_2CH_3$ | H | $C_2H_5$ | H | 0 | H | H | H | H | |
| 1.045 | $CO_2CH_3$ | H | $C_2H_5$ | H | 0 | H | H | H | H | 3,4-cis Smp.101-103°C |
| 1.046 | $CO_2CH_3$ | H | $C_2H_5$ | H | 0 | H | H | H | H | 3,4-trans |
| 1.047 | $CO_2CH_3$ | H | $C_2H_5$ | $CH_3$ | 0 | H | H | H | H | |
| 1.048 | $CO_2CH_3$ | H | $C_2H_5$ | $C_2H_5$ | 0 | H | H | H | H | |
| 1.049 | $CO_2CH_3$ | SH | $C_2H_5$ | $C_2H_5$ | 0 | H | H | H | H | |
| 1.050 | $CO_2CH_3$ | H | $C_2H_5$ | $C_2H_5$ | 0 | =O | | H | H | |
| 1.051 | $CO_2CH_3$ | H | $C_2H_5$ | $C_2H_5$ | 0 | H | H | 6-$CH_3$ | H | |
| 1.052 | $CO_2CH_3$ | H | $C_2H_5$ | $C_2H_5$ | 0 | H | H | 6-F | H | |
| 1.053 | $CO_2CH_3$ | H | $C_2H_5$ | $C_2H_5$ | 0 | H | H | 5-$CH_3$ | H | |
| 1.054 | $CO_2CH_3$ | SH | $C_2H_5$ | $C_2H_5$ | 0 | H | H | 5-$CH_3$ | H | |
| 1.055 | $CO_2CH_3$ | H | $C_2H_5$ | $C_2H_5$ | 0 | $CH_3$ | H | H | H | |
| 1.056 | $CO_2CH_3$ | H | $C_2H_5$ | H | 0 | $CH_3$ | H | H | H | |
| 1.057 | $CO_2CH_3$ | H | $C_2H_5$ | H | 0 | $C_2H_5$ | H | H | H | |
| 1.058 | $CO_2CH_3$ | H | $C_2H_5$ | H | 0 | $C_2H_5$ | H | 6-$CH_3$ | H | |
| 1.059 | $CO_2CH_3$ | H | $C_2H_5$ | $C_2H_5$ | 0 | $C_2H_5$ | $CH_3$ | H | H | |
| 1.060 | $CO_2CH_3$ | H | $C_2H_5$ | $C_2H_5$ | 0 | $CH_3$ | $CH_3$ | H | H | |
| 1.061 | $CO_2CH_3$ | H | $C_3H_7$-n | H | 0 | H | H | H | H | |
| 1.062 | $CO_2CH_3$ | H | $C_3H_7$-i | H | 0 | H | H | H | H | |
| 1.063 | $CO_2CH_3$ | H | $C_3H_7$-i | H | 0 | $CH_3$ | H | H | H | |
| 1.064 | $CO_2CH_3$ | H | $-(CH_2)_4-$ | | 0 | H | H | H | H | |
| 1.065 | $CO_2CH_3$ | H | $-(CH_2)_4-$ | | 0 | $CH_3$ | H | H | H | |
| 1.066 | $CO_2CH_3$ | SH | $-(CH_2)_4-$ | | 0 | H | H | H | H | |
| 1.067 | $CO_2CH_3$ | H | $CH_2CH=CH_2$ | H | 0 | H | H | H | H | |
| 1.068 | $CO_2CH_3$ | H | $CH_2CH=CH_2$ | $CH_3$ | 0 | H | H | H | H | |
| 1.069 | $CO_2CH_3$ | H | $C_4H_9$-n | H | 0 | H | H | H | H | |
| 1.070 | $CO_2CH_3$ | H | $CH_2$ | H | 0 | H | H | H | H | |
| 1.071 | $CO_2C_2H_5$ | H | $CH_3$ | H | 0 | H | H | H | H | |
| 1.072 | $CO_2C_2H_5$ | H | $CH_3$ | H | 0 | H | H | H | H | 3,4-cis |
| 1.073 | $CO_2C_2H_5$ | H | $CH_3$ | H | 0 | H | H | H | H | 3,4-trans |
| 1.074 | $CO_2C_2H_5$ | SH | $CH_3$ | H | 0 | $CH_3$ | H | H | H | |
| 1.075 | $CO_2C_2H_5$ | H | $CH_3$ | H | 0 | =O | | H | H | |
| 1.076 | $CO_2C_2H_5$ | H | $CH_3$ | H | 0 | $CH_3$ | H | H | H | |
| 1.077 | $CO_2C_2H_5$ | H | $CH_3$ | H | 0 | H | H | 5-$CH_3$ | H | |
| 1.078 | $CO_2C_2H_5$ | H | $CH_3$ | H | 0 | H | H | 6-$CH_3$ | H | |
| 1.079 | $CO_2C_2H_5$ | H | $CH_3$ | $CH_3$ | 0 | H | H | H | H | |
| 1.080 | $CO_2C_2H_5$ | H | $CH_3$ | $CH_3$ | 0 | $CH_3$ | H | H | H | |
| 1.081 | $CO_2C_2H_5$ | SH | $CH_3$ | $CH_3$ | 0 | H | H | H | H | |
| 1.082 | $CO_2C_2H_5$ | H | $CH_3$ | $CH_3$ | 0 | =O | | H | H | |
| 1.083 | $CO_2C_2H_5$ | H | $CH_3$ | $CH_3$ | 0 | H | H | 5-$CH_3$ | H | |
| 1.084 | $CO_2C_2H_5$ | H | $CH_3$ | $CH_3$ | 0 | H | H | 5-F | H | |
| 1.085 | $CO_2C_2H_5$ | H | $CH_3$ | $CH_3$ | 0 | H | H | 6-$CH_3$ | H | |

Fortsetzung Tabelle 1:

| Verb. Nr. | L | $R^1$ | $R^2$ | $R^3$ | n | $R^4$ | $R^5$ | $R^6$ | $R^7$ | phys. Daten |
|---|---|---|---|---|---|---|---|---|---|---|
| 1.086 | $CO_2C_2H_5$ | SH | $-CH_2-CH_2-$ | | 0 | H | H | H | H | |
| 1.087 | $CO_2C_2H_5$ | SH | $-CH_2-CH_2-$ | | 0 | H | H | H | H | |
| 1.088 | $CO_2C_2H_5$ | H | $C_2H_5$ | H | 0 | H | H | H | H | |
| 1.089 | $CO_2C_2H_5$ | H | $C_2H_5$ | H | 0 | H | H | H | H | 3,4-cis |
| 1.090 | $CO_2C_2H_5$ | H | $C_2H_5$ | H | 0 | H | H | H | H | 3,4-trans |
| 1.091 | $CO_2C_2H_5$ | H | $C_2H_5$ | H | 0 | $CH_3$ | H | H | H | |
| 1.092 | $CO_2C_2H_5$ | H | $C_2H_5$ | $C_2H_5$ | 0 | H | H | H | H | |
| 1.093 | $CO_2C_2H_5$ | H | $C_2H_5$ | $C_2H_5$ | 0 | $CH_3$ | H | H | H | |
| 1.094 | $CO_2C_2H_5$ | SH | $C_2H_5$ | $C_2H_5$ | 0 | H | H | H | H | |
| 1.095 | $CO_2C_2H_5$ | H | $C_2H_5$ | $C_2H_5$ | 0 | $C_2H_5$ | H | H | H | |
| 1.096 | $CO_2C_2H_5$ | H | $C_2H_5$ | $C_2H_5$ | 0 | =O | | H | H | |
| 1.097 | $COCH_3$ | H | $CH_3$ | H | 0 | H | H | H | H | |
| 1.098 | $COCH_3$ | H | $CH_3$ | H | 0 | H | H | H | H | 3,4-cis |
| 1.099 | $COCH_3$ | H | $CH_3$ | H | 0 | H | H | H | H | 3,4-trans |
| 1.100 | $COCH_3$ | SH | $CH_3$ | H | 0 | H | H | H | H | |
| 1.101 | $COCH_3$ | H | $CH_3$ | H | 0 | $CH_3$ | H | H | H | |
| 1.102 | $COCH_3$ | H | $CH_3$ | $CH_3$ | 0 | H | H | H | H | |
| 1.103 | $COCH_3$ | SH | $CH_3$ | $CH_3$ | 0 | H | H | H | H | |
| 1.104 | $COCH_3$ | H | $-CH_2-CH_2-$ | | 0 | H | H | H | H | |
| 1.105 | $COCH_3$ | H | $C_2H_5$ | H | 0 | H | H | H | H | |
| 1.106 | $COCH_3$ | H | $C_2H_5$ | $C_2H_5$ | 0 | H | H | H | H | |
| 1.107 | $COCH_3$ | H | $C_2H_5$ | H | 0 | $CH_3$ | H | H | H | |
| 1.108 | $COCH_3$ | H | $C_2H_5$ | $C_2H_5$ | 0 | =O | | H | H | |
| 1.109 | $COC_2H_5$ | H | $CH_3$ | H | 0 | H | H | H | H | |
| 1.110 | $COC_2H_5$ | H | $CH_3$ | $CH_3$ | 0 | H | H | H | H | |
| 1.111 | $COC_2H_5$ | H | $CH_3$ | $CH_3$ | 0 | $CH_3$ | H | H | H | |
| 1.112 | $COC_2H_5$ | H | $-CH_2-CH_2-$ | | 0 | $CH_3$ | H | H | H | |
| 1.113 | $COC_2H_5$ | H | $C_2H_5$ | H | 0 | H | H | H | H | |
| 1.114 | $COC_2H_5$ | H | $C_2H_5$ | $C_2H_5$ | 0 | H | H | H | H | |
| 1.115 | $CONH_2$ | H | $CH_3$ | H | 0 | H | H | H | H | |
| 1.116 | $CONH_2$ | H | $CH_3$ | $CH_3$ | 0 | H | H | H | H | |
| 1.117 | $CONH_2$ | H | $-CH_2-CH_2-$ | | 0 | H | H | H | H | |
| 1.118 | $CONH_2$ | H | $C_2H_5$ | H | 0 | H | H | H | H | |
| 1.119 | $CONH_2$ | H | $C_2H_5$ | $C_2H_5$ | 0 | H | H | H | H | |
| 1.120 | $CONHCH_3$ | H | $CH_3$ | H | 0 | H | H | H | H | |
| 1.121 | $CONHCH_3$ | H | $CH_3$ | $CH_3$ | 0 | H | H | H | H | |
| 1.122 | $CONHCH_3$ | H | $-CH_2-CH_2-$ | | 0 | H | H | H | H | |
| 1.123 | $CONHCH_3$ | H | $C_2H_5$ | H | 0 | H | H | H | H | |
| 1.124 | $CONHCH_3$ | H | $C_2H_5$ | $C_2H_5$ | 0 | H | H | H | H | |
| 1.125 | $CON(CH_3)_2$ | H | $CH_3$ | H | 0 | H | H | H | H | |
| 1.126 | $CON(CH_3)_2$ | H | $CH_3$ | $CH_3$ | 0 | H | H | H | H | |
| 1.127 | $CON(CH_3)_2$ | H | $-CH_2-CH_2-$ | | 0 | H | H | H | H | |
| 1.128 | $CON(CH_3)_2$ | H | $C_2H_5$ | H | 0 | H | H | H | H | |
| 1.129 | $CON(CH_3)_2$ | H | $C_2H_5$ | $C_2H_5$ | 0 | H | H | H | H | |
| 1.130 | CN | H | $CH_3$ | $CH_3$ | 0 | H | H | H | H | |
| 1.131 | $CO_2CH_2CH=CH_2$ | H | $CH_3$ | $CH_3$ | 0 | H | H | H | H | |
| 1.132 | $CO_2CH_2C\equiv CH$ | H | $CH_3$ | $CH_3$ | 0 | H | H | H | H | |
| 1.133 | $CO_2C_3H_7$ | H | $CH_3$ | $CH_3$ | 0 | H | H | H | H | |

Fortsetzung Tabelle 1:

| Verb. Nr. | L | $R^1$ | $R^2$ | $R^3$ | n | $R^4$ | $R^5$ | $R^6$ | $R^7$ | phys. Daten |
|---|---|---|---|---|---|---|---|---|---|---|
| 1.134 | $CONHOCH_3$ | H | $CH_3$ | $CH_3$ | 0 | H | H | H | H | |
| 1.135 | $CO_2CH_3$ | H | $CH_3$ | H | 1 | H | H | H | H | |
| 1.136 | $CO_2CH_3$ | H | $CH_3$ | H | 1 | H | H | H | H | 2,3-cis |
| 1.137 | $CO_2CH_3$ | H | $CH_3$ | H | 1 | H | H | H | H | 2,3-trans |
| 1.138 | $CO_2CH_3$ | H | $CH_3$ | $CH_3$ | 1 | H | H | H | H | |
| 1.139 | $CO_2CH_3$ | H | $-CH_2-CH_2-$ | | 1 | H | H | H | H | |
| 1.140 | $CO_2CH_3$ | H | $C_2H_5$ | H | 1 | H | H | H | H | |
| 1.141 | $CO_2CH_3$ | H | $C_2H_5$ | $C_2H_5$ | 1 | H | H | H | H | |
| 1.142 | $CO_2C_2H_5$ | H | $CH_3$ | $CH_3$ | 1 | H | H | H | H | |
| 1.143 | $COCH_3$ | H | $CH_3$ | $CH_3$ | 1 | H | H | H | H | |
| 1.144 | $CO_2C_2H_5$ | H | $CH_3$ | $CH_3$ | 1 | H | H | H | H | |
| 1.145 | $CONHCH_3$ | H | $CH_3$ | $CH_3$ | 1 | H | H | H | H | |
| 1.146 | $CON(CH_3)_2$ | H | $CH_3$ | $CH_3$ | 1 | H | H | H | H | |
| 1.147 | CN | H | $CH_3$ | $CH_3$ | 1 | H | H | H | H | |
| 1.148 | $CO_2CH_3$ | H | $CH_3$ | H | 2 | H | H | H | H | |
| 1.149 | $CO_2CH_3$ | H | $CH_3$ | H | 2 | H | H | H | H | 3,4-cis |
| 1.150 | $CO_2CH_3$ | H | $CH_3$ | H | 2 | H | H | H | H | 3,4-trans |
| 1.151 | $CO_2CH_3$ | H | $CH_3$ | $CH_3$ | 2 | H | H | H | H | Smp.171-173°C |
| 1.152 | $CO_2CH_3$ | H | $C_2H_5$ | H | 2 | H | H | H | H | |
| 1.153 | $CO_2CH_3$ | H | $C_2H_5$ | $C_2H_5$ | 2 | H | H | H | H | |
| 1.154 | $CO_2CH_3$ | H | H | H | 2 | $CH_3$ | H | H | H | |
| 1.155 | $CO_2C_2H_5$ | H | $CH_3$ | $CH_3$ | 2 | H | H | H | H | |
| 1.156 | $COCH_3$ | H | $CH_3$ | $CH_3$ | 2 | H | H | H | H | |
| 1.157 | $COC_2H_5$ | H | $CH_3$ | $CH_3$ | 2 | H | H | H | H | |
| 1.158 | $CONHCH_3$ | H | $CH_3$ | $CH_3$ | 2 | H | H | H | H | |
| 1.159 | $CON(CH_3)_2$ | H | $CH_3$ | $CH_3$ | 2 | H | H | H | H | |
| 1.160 | CN | H | $CH_3$ | $CH_3$ | 2 | H | H | H | H | |

Tabelle 2:

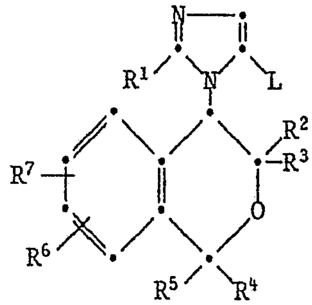

| Verb. Nr. | L | $R^1$ | $R^2$ | $R^3$ | $R^4$ | $R^5$ | $R^6$ | $R^7$ | phys. Daten |
|---|---|---|---|---|---|---|---|---|---|
| 2.001 | $CO_2CH_3$ | H | H | H | H | H | H | H | Smp. 48-50°C |
| 2.002 | $CO_2CH_3$ | SH | H | H | H | H | H | H | |
| 2.003 | $CO_2CH_3$ | H | $CH_3$ | H | H | H | H | H | |
| 2.004 | $CO_2CH_3$ | H | $CH_3$ | H | H | H | H | H | 3,4-cis |
| 2.005 | $CO_2CH_3$ | H | $CH_3$ | H | H | H | H | H | 3,4-trans |
| 2.006 | $CO_2CH_3$ | H | $CH_3$ | H | =O | | H | H | |
| 2.007 | $CO_2CH_3$ | H | $CH_3$ | H | $CH_3$ | H | H | H | |
| 2.008 | $CO_2CH_3$ | H | $CH_3$ | H | $CH_3$ | $CH_3$ | H | H | |
| 2.009 | $CO_2CH_3$ | H | $CH_3$ | H | $CH_3$ | H | 5-$CH_3$ | H | |
| 2.010 | $CO_2CH_3$ | H | $CH_3$ | H | $CH_3$ | H | 5-F | H | |
| 2.011 | $CO_2CH_3$ | SH | $CH_3$ | H | $CH_3$ | H | H | H | |
| 2.012 | $CO_2CH_3$ | H | $CH_3$ | H | $CH_3$ | H | 6-$CH_3$ | H | |
| 2.013 | $CO_2CH_3$ | H | $CH_3$ | H | $CH_3$ | H | 6-$OCH_3$ | H | |
| 2.014 | $CO_2CH_3$ | H | $CH_3$ | H | $CH_3$ | H | 5-$CH_3$ | 8-$CH_3$ | |
| 2.015 | $CO_2CH_3$ | H | $CH_3$ | H | $C_2H_5$ | H | H | H | |
| 2.016 | $CO_2CH_3$ | H | $CH_3$ | $CH_3$ | H | H | H | H | Smp.110-112°C |
| 2.017 | $CO_2CH_3$ | SH | $CH_3$ | $CH_3$ | H | H | H | H | |
| 2.018 | $CO_2CH_3$ | H | $CH_3$ | $CH_3$ | $CH_3$ | H | H | H | |
| 2.019 | $CO_2CH_3$ | H | $CH_3$ | $CH_3$ | $CH_3$ | H | H | H | 1,4-cis |
| 2.020 | $CO_2CH_3$ | H | $CH_3$ | $CH_3$ | $CH_3$ | H | H | H | 1,4-trans |
| 2.021 | $CO_2CH_3$ | H | $CH_3$ | $CH_3$ | $CH_3$ | $CH_3$ | H | H | |
| 2.022 | $CO_2CH_3$ | H | $CH_3$ | $CH_3$ | $CH_3$ | H | 6-$CH_3$ | H | |
| 2.023 | $CO_2CH_3$ | H | $CH_3$ | $CH_3$ | H | H | 6-$CH_3$ | H | |
| 2.024 | $CO_2CH_3$ | H | $CH_3$ | $CH_3$ | H | H | 5-$CH_3$ | 8-$CH_3$ | |
| 2.025 | $CO_2CH_3$ | H | $CH_3$ | $CH_3$ | H | H | 7-$OCH_3$ | H | |
| 2.026 | $CO_2CH_3$ | H | $CH_3$ | $CH_3$ | H | H | 6-$OCH_3$ | H | |
| 2.027 | $CO_2CH_3$ | H | $CH_3$ | $CH_3$ | H | H | 6-F | H | |
| 2.028 | $CO_2CH_3$ | H | $CH_3$ | $CH_3$ | H | H | 6-Cl | H | |
| 2.029 | $CO_2CH_3$ | H | $CH_3$ | $CH_3$ | H | H | 6-Br | H | |
| 2.030 | $CO_2CH_3$ | H | $CH_3$ | $CH_3$ | H | H | 6-$NO_2$ | H | |
| 2.031 | $CO_2CH_3$ | H | $CH_3$ | $CH_3$ | H | H | 5-F | H | |
| 2.032 | $CO_2CH_3$ | H | $CH_3$ | $CH_3$ | H | H | 5-$CH_3$ | H | |
| 2.033 | $CO_2CH_3$ | H | $CH_3$ | $CH_3$ | H | H | 5-$OCH_3$ | H | |
| 2.034 | $CO_2CH_3$ | H | $CH_3$ | $CH_3$ | =O | | H | H | Smp.123-125°C |
| 2.035 | $CO_2CH_3$ | H | $-CH_2-CH_2-$ | | H | H | H | H | |
| 2.036 | $CO_2CH_3$ | SH | $-CH_2-CH_2-$ | | H | H | H | H | |
| 2.037 | $CO_2CH_3$ | H | $-CH_2-CH_2-$ | | $CH_3$ | H | H | H | |
| 2.038 | $CO_2CH_3$ | H | H | H | $CH_3$ | H | H | H | |

Fortsetzung Tabelle 2:

| Verb. Nr. | L | $R^1$ | $R^2$ | $R^3$ | $R^4$ | $R^5$ | $R^6$ | $R^7$ | phys. Daten |
|---|---|---|---|---|---|---|---|---|---|
| 2.039 | $CO_2CH_3$ | H | H | H | $CH_3$ | H | H | H | 1,4-cis |
| 2.040 | $CO_2CH_3$ | H | H | H | $CH_3$ | H | H | H | 1,4-trans |
| 2.041 | $CO_2CH_3$ | H | H | H | $CH_3$ | $CH_3$ | H | H | |
| 2.042 | $CO_2CH_3$ | SH | H | H | $CH_3$ | $CH_3$ | H | H | |
| 2.043 | $CO_2CH_3$ | H | H | H | $C_2H_5$ | H | H | H | |
| 2.044 | $CO_2CH_3$ | H | $C_2H_5$ | H | H | H | H | H | |
| 2.045 | $CO_2CH_3$ | H | $C_2H_5$ | H | H | H | H | H | 3,4-cis |
| 2.046 | $CO_2CH_3$ | H | $C_2H_5$ | H | H | H | H | H | 3,4-trans |
| 2.047 | $CO_2CH_3$ | H | $C_2H_5$ | $CH_3$ | H | H | H | H | |
| 2.048 | $CO_2CH_3$ | H | $C_2H_5$ | $C_2H_5$ | H | H | H | H | |
| 2.049 | $CO_2CH_3$ | SH | $C_2H_5$ | $C_2H_5$ | H | H | H | H | |
| 2.050 | $CO_2CH_3$ | H | $C_2H_5$ | $C_2H_5$ | =O | | H | H | |
| 2.051 | $CO_2CH_3$ | H | $C_2H_5$ | $C_2H_5$ | H | H | $6-CH_3$ | H | |
| 2.052 | $CO_2CH_3$ | H | $C_2H_5$ | $C_2H_5$ | H | H | 6-F | H | |
| 2.053 | $CO_2CH_3$ | H | $C_2H_5$ | $C_2H_5$ | H | H | $5-CH_3$ | H | |
| 2.054 | $CO_2CH_3$ | SH | $C_2H_5$ | $C_2H_5$ | H | H | $5-CH_3$ | H | |
| 2.055 | $CO_2CH_3$ | H | $C_2H_5$ | $C_2H_5$ | $CH_3$ | H | H | H | |
| 2.056 | $CO_2CH_3$ | H | $C_2H_5$ | H | $CH_3$ | H | H | H | |
| 2.057 | $CO_2CH_3$ | H | $C_2H_5$ | H | $C_2H_5$ | H | H | H | |
| 2.058 | $CO_2CH_3$ | H | $C_2H_5$ | H | $C_2H_5$ | H | $6-CH_3$ | H | |
| 2.059 | $CO_2CH_3$ | H | $C_2H_5$ | $C_2H_5$ | $C_2H_5$ | $CH_3$ | H | H | |
| 2.060 | $CO_2CH_3$ | H | $C_2H_5$ | $C_2H_5$ | $CH_3$ | $CH_3$ | H | H | |
| 2.061 | $CO_2CH_3$ | H | $C_3H_7$-n | H | H | H | H | H | |
| 2.062 | $CO_2CH_3$ | H | $C_3H_7$-i | H | H | H | H | H | |
| 2.063 | $CO_2CH_3$ | H | $C_3H_7$-i | H | $CH_3$ | H | H | H | |
| 2.064 | $CO_2CH_3$ | H | $-(CH_2)_4-$ | | H | H | H | H | |
| 2.065 | $CO_2CH_3$ | H | $-(CH_2)_4-$ | | $CH_3$ | H | H | H | |
| 2.066 | $CO_2CH_3$ | SH | $-(CH_2)_4-$ | | H | H | H | H | |
| 2.067 | $CO_2CH_3$ | H | $CH_2CH=CH_2$ | H | H | H | H | H | |
| 2.068 | $CO_2CH_3$ | H | $CH_2CH=CH_2$ | $CH_3$ | H | H | H | H | |
| 2.069 | $CO_2CH_3$ | H | $C_4H_9$-n | H | H | H | H | H | |
| 2.070 | $CO_2CH_3$ | H | $CH_2$ | H | H | H | H | H | |
| 2.071 | $CO_2C_2H_5$ | H | $CH_3$ | H | H | H | H | H | |
| 2.072 | $CO_2C_2H_5$ | H | $CH_3$ | H | H | H | H | H | 3,4-cis |
| 2.073 | $CO_2C_2H_5$ | H | $CH_3$ | H | H | H | H | H | 3,4-trans |
| 2.074 | $CO_2C_2H_5$ | SH | $CH_3$ | H | $CH_3$ | H | H | H | |
| 2.075 | $CO_2C_2H_5$ | H | $CH_3$ | H | =O | | H | H | |
| 2.076 | $CO_2C_2H_5$ | H | $CH_3$ | H | $CH_3$ | H | H | H | |
| 2.077 | $CO_2C_2H_5$ | H | $CH_3$ | H | H | H | $5-CH_3$ | H | |
| 2.078 | $CO_2C_2H_5$ | H | $CH_3$ | H | H | H | $6-CH_3$ | H | |
| 2.079 | $CO_2C_2H_5$ | H | $CH_3$ | $CH_3$ | H | H | H | H | |
| 2.080 | $CO_2C_2H_5$ | H | $CH_3$ | $CH_3$ | $CH_3$ | H | H | H | |
| 2.081 | $CO_2C_2H_5$ | SH | $CH_3$ | $CH_3$ | H | H | H | H | |
| 2.082 | $CO_2C_2H_5$ | H | $CH_3$ | $CH_3$ | =O | | H | H | |
| 2.083 | $CO_2C_2H_5$ | H | $CH_3$ | $CH_3$ | H | H | $5-CH_3$ | H | |
| 2.084 | $CO_2C_2H_5$ | H | $CH_3$ | $CH_3$ | H | H | 5-F | H | |
| 2.085 | $CO_2C_2H_5$ | H | $CH_3$ | $CH_3$ | H | H | $6-CH_3$ | H | |
| 2.086 | $CO_2C_2H_5$ | SH | $-CH_2-CH_2-$ | | H | H | H | H | |

Fortsetzung Tabelle 2:

| Verb. Nr. | L | $R^1$ | $R^2$ | $R^3$ | $R^4$ | $R^5$ | $R^6$ | $R^7$ | phys. Daten |
|---|---|---|---|---|---|---|---|---|---|
| 2.087 | $CO_2C_2H_5$ | SH | $-CH_2-CH_2-$ | | H | H | H | H | |
| 2.088 | $CO_2C_2H_5$ | H | $C_2H_5$ | H | H | H | H | H | |
| 2.089 | $CO_2C_2H_5$ | H | $C_2H_5$ | H | H | H | H | H | 3,4-cis |
| 2.090 | $CO_2C_2H_5$ | H | $C_2H_5$ | H | H | H | H | H | 3,4-trans |
| 2.091 | $CO_2C_2H_5$ | H | $C_2H_5$ | H | $CH_3$ | H | H | H | |
| 2.092 | $CO_2C_2H_5$ | H | $C_2H_5$ | $C_2H_5$ | H | H | H | H | |
| 2.093 | $CO_2C_2H_5$ | H | $C_2H_5$ | $C_2H_5$ | $CH_3$ | H | H | H | |
| 2.094 | $CO_2C_2H_5$ | SH | $C_2H_5$ | $C_2H_5$ | H | H | H | H | |
| 2.095 | $CO_2C_2H_5$ | H | $C_2H_5$ | $C_2H_5$ | $C_2H_5$ | H | H | H | |
| 2.096 | $CO_2C_2H_5$ | H | $C_2H_5$ | $C_2H_5$ | =O | | H | H | |
| 2.097 | $COCH_3$ | H | $CH_3$ | H | H | H | H | H | |
| 2.098 | $COCH_3$ | H | $CH_3$ | H | H | H | H | H | 3,4-cis |
| 2.099 | $COCH_3$ | H | $CH_3$ | H | H | H | H | H | 3,4-trans |
| 2.100 | $COCH_3$ | SH | $CH_3$ | H | H | H | H | H | |
| 2.101 | $COCH_3$ | H | $CH_3$ | H | $CH_3$ | H | H | H | |
| 2.102 | $COCH_3$ | H | $CH_3$ | $CH_3$ | H | H | H | H | |
| 2.103 | $COCH_3$ | SH | $CH_3$ | $CH_3$ | H | H | H | H | |
| 2.104 | $COCH_3$ | H | $-CH_2-CH_2-$ | | H | H | H | H | |
| 2.105 | $COCH_3$ | H | $C_2H_5$ | H | H | H | H | H | |
| 2.106 | $COCH_3$ | H | $C_2H_5$ | $C_2H_5$ | H | H | H | H | |
| 2.107 | $COCH_3$ | H | $C_2H_5$ | H | $CH_3$ | H | H | H | |
| 2.108 | $COCH_3$ | H | $C_2H_5$ | $C_2H_5$ | =O | | H | H | |
| 2.109 | $COC_2H_5$ | H | $CH_3$ | H | H | H | H | H | |
| 2.110 | $COC_2H_5$ | H | $CH_3$ | $CH_3$ | H | H | H | H | |
| 2.111 | $COC_2H_5$ | H | $CH_3$ | $CH_3$ | $CH_3$ | H | H | H | |
| 2.112 | $COC_2H_5$ | H | $-CH_2-CH_2-$ | | $CH_3$ | H | H | H | |
| 2.113 | $COC_2H_5$ | H | $C_2H_5$ | H | H | H | H | H | |
| 2.114 | $COC_2H_5$ | H | $C_2H_5$ | $C_2H_5$ | H | H | H | H | |
| 2.115 | $CONH_2$ | H | $CH_3$ | H | H | H | H | H | |
| 2.116 | $CONH_2$ | H | $CH_3$ | $CH_3$ | H | H | H | H | |
| 2.117 | $CONH_2$ | H | $-CH_2-CH_2-$ | | H | H | H | H | |
| 2.118 | $CONH_2$ | H | $C_2H_5$ | H | H | H | H | H | |
| 2.119 | $CONH_2$ | H | $C_2H_5$ | $C_2H_5$ | H | H | H | H | |
| 2.120 | $CONHCH_3$ | H | $CH_3$ | H | H | H | H | H | |
| 2.121 | $CONHCH_3$ | H | $CH_3$ | $CH_3$ | H | H | H | H | |
| 2.122 | $CONHCH_3$ | H | $-CH_2-CH_2-$ | | H | H | H | H | |
| 2.123 | $CONHCH_3$ | H | $C_2H_5$ | H | H | H | H | H | |
| 2.124 | $CONHCH_3$ | H | $C_2H_5$ | $C_2H_5$ | H | H | H | H | |
| 2.125 | $CON(CH_3)_2$ | H | $CH_3$ | H | H | H | H | H | |
| 2.126 | $CON(CH_3)_2$ | H | $CH_3$ | $CH_3$ | H | H | H | H | |
| 2.127 | $CON(CH_3)_2$ | H | $-CH_2-CH_2-$ | | H | H | H | H | |
| 2.128 | $CON(CH_3)_2$ | H | $C_2H_5$ | H | H | H | H | H | |
| 2.129 | $CON(CH_3)_2$ | H | $C_2H_5$ | $C_2H_5$ | H | H | H | H | |
| 2.130 | CN | H | $CH_3$ | $CH_3$ | H | H | H | H | |
| 2.131 | $CO_2CH_2CH=CH_2$ | H | $CH_3$ | $CH_3$ | H | H | H | H | |
| 2.132 | $CO_2CH_2C\equiv CH$ | H | $CH_3$ | $CH_3$ | H | H | H | H | |
| 2.133 | $CH_2-OH$ | H | $CH_3$ | $CH_3$ | H | H | H | H | Smp.159-161°C |

Tabelle 3:

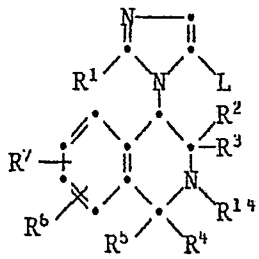

| Verb. Nr. | L | $R^1$ | $R^2$ | $R^3$ | $R^{14}$ | $R^4$ | $R^5$ | $R^6$ | $R^7$ | phys. Daten |
|---|---|---|---|---|---|---|---|---|---|---|
| 3.01 | $CO_2CH_3$ | H | H | H | H | H | H | H | H | |
| 3.02 | $CO_2CH_3$ | H | H | H | H | =O | | H | H | |
| 3.03 | $CO_2CH_3$ | H | H | H | $CH_3$ | H | H | H | H | $n_D^{23}$:1,5606 |
| 3.04 | $CO_2CH_3$ | H | H | H | Benzyl | H | H | H | H | |
| 3.05 | $CO_2CH_3$ | H | $CH_3$ | H | H | H | H | H | H | |
| 3.06 | $CO_2CH_3$ | H | $CH_3$ | H | H | H | H | H | H | 3,4-cis |
| 3.07 | $CO_2CH_3$ | H | $CH_3$ | H | H | H | H | H | H | 3,4-trans |
| 3.08 | $CO_2CH_3$ | H | $CH_3$ | H | $CH_3$ | H | H | H | H | |
| 3.09 | $CO_2CH_3$ | H | $CH_3$ | H | $CH_3$ | H | $CH_3$ | H | H | |
| 3.10 | $CO_2CH_3$ | H | $CH_3$ | H | H | H | $CH_3$ | H | H | |
| 3.11 | $CO_2CH_3$ | H | $CH_3$ | H | H | H | H | 6-$CH_3$ | H | |
| 3.12 | $CO_2CH_3$ | H | $CH_3$ | H | H | H | H | 6-$OCH_3$ | H | |
| 3.13 | $CO_2CH_3$ | H | $CH_3$ | H | H | H | H | 6-$OCH_3$ | 7-$OCH_3$ | |
| 3.14 | $CO_2CH_3$ | SH | $CH_3$ | H | $CH_3$ | H | H | H | H | |
| 3.15 | $CO_2CH_3$ | SH | $CH_3$ | $CH_3$ | H | H | H | H | H | |
| 3.16 | $CO_2CH_3$ | H | $CH_3$ | $CH_3$ | H | H | H | H | H | |
| 3.17 | $CO_2CH_3$ | H | $CH_3$ | $CH_3$ | H | =O | | H | H | |
| 3.18 | $CO_2CH_3$ | SH | $CH_3$ | $CH_3$ | $CH_3$ | H | H | H | H | |
| 3.19 | $CO_2CH_3$ | H | $CH_3$ | $CH_3$ | $CH_3$ | H | H | H | H | |
| 3.20 | $CO_2CH_3$ | H | $CH_3$ | $CH_3$ | $CH_3$ | =O | | H | H | |
| 3.21 | $CO_2CH_3$ | H | $CH_3$ | $CH_3$ | H | H | $CH_3$ | H | H | |
| 3.22 | $CO_2CH_3$ | H | $CH_3$ | $CH_3$ | $CH_3$ | H | H | 6-$CH_3$ | H | |
| 3.23 | $CO_2CH_3$ | H | $CH_3$ | $CH_3$ | H | H | H | 6-$OCH_3$ | 7-$OCH_3$ | |
| 3.24 | $CO_2CH_3$ | H | $C_2H_5$ | H | H | H | H | H | H | |
| 3.25 | $CO_2CH_3$ | H | $C_2H_5$ | H | $CH_3$ | H | H | H | H | |
| 3.26 | $CO_2CH_3$ | H | $C_2H_5$ | H | H | H | $CH_3$ | H | H | |
| 3.27 | $CO_2CH_3$ | H | $C_2H_5$ | $C_2H_5$ | H | H | H | H | H | |
| 3.28 | $CO_2CH_3$ | H | $C_2H_5$ | $C_2H_5$ | H | =O | | H | H | |
| 3.29 | $CO_2CH_3$ | H | $C_2H_5$ | $C_2H_5$ | $CH_3$ | H | H | H | H | |
| 3.30 | $CO_2CH_3$ | H | $C_2H_5$ | $C_2H_5$ | H | H | $CH_3$ | H | H | |
| 3.31 | $CO_2CH_3$ | SH | $C_2H_5$ | $C_2H_5$ | $CH_3$ | H | H | H | H | |
| 3.32 | $CO_2CH_3$ | H | $-CH_2-CH_2-$ | | $CH_3$ | H | H | H | H | |
| 3.33 | $CO_2C_2H_5$ | H | H | H | H | H | H | H | H | |
| 3.34 | $CO_2C_2H_5$ | H | H | H | $CH_3$ | H | H | H | H | |
| 3.35 | $CO_2C_2H_5$ | H | $CH_3$ | H | H | H | H | H | H | |
| 3.36 | $CO_2C_2H_5$ | H | $CH_3$ | H | $CH_3$ | H | H | H | H | |
| 3.37 | $CO_2C_2H_5$ | H | $CH_3$ | H | $CH_3$ | H | $CH_3$ | H | H | |
| 3.38 | $CO_2C_2H_5$ | H | $CH_3$ | H | $CH_3$ | H | H | 6-$CH_3$ | H | |
| 3.39 | $CO_2C_2H_5$ | H | $CH_3$ | $CH_3$ | H | H | H | H | H | |
| 3.40 | $CO_2C_2H_5$ | H | $CH_3$ | $CH_3$ | H | =O | | H | H | |

Fortsetzung Tabelle 3:

| Verb. Nr. | L | $R^1$ | $R^2$ | $R^3$ | $R^{14}$ | $R^4$ | $R^5$ | $R^6$ | $R^7$ | phys. Daten |
|---|---|---|---|---|---|---|---|---|---|---|
| 3.41 | $CO_2C_2H_5$ | H | $CH_3$ | $CH_3$ | $CH_3$ | H | H | H | H | |
| 3.42 | $CO_2C_2H_5$ | SH | $CH_3$ | $CH_3$ | $CH_3$ | H | H | H | H | |
| 3.43 | $CO_2C_2H_5$ | H | $CH_3$ | $CH_3$ | $CH_3$ | H | H | 6-$OCH_3$ | H | |
| 3.44 | $CO_2C_2H_5$ | H | $CH_3$ | $CH_3$ | $CH_3$ | H | H | 6-$OCH_3$ | 7-$OCH_3$ | |
| 3.45 | $CO_2C_2H_5$ | H | $C_2H_5$ | H | H | H | H | H | H | |
| 3.46 | $CO_2C_2H_5$ | H | $C_2H_5$ | H | $CH_3$ | H | H | H | H | |
| 3.47 | $CO_2C_2H_5$ | H | $C_2H_5$ | $C_2H_5$ | H | H | H | H | H | |
| 3.48 | $CO_2C_2H_5$ | H | $C_2H_5$ | $C_2H_5$ | $CH_3$ | H | H | H | H | |
| 3.49 | $CO_2C_2H_5$ | SH | $C_2H_5$ | $C_2H_5$ | $CH_3$ | H | H | H | H | |
| 3.50 | $CO_2C_2H_5$ | H | $-CH_2-CH_2-$ | | $CH_3$ | H | H | H | H | |
| 3.51 | $COCH_3$ | H | H | H | H | H | H | H | H | |
| 3.52 | $COCH_3$ | SH | H | H | $CH_3$ | H | H | H | H | |
| 3.53 | $COCH_3$ | H | $CH_3$ | H | $CH_3$ | H | H | H | H | |
| 3.54 | $COCH_3$ | H | $CH_3$ | $CH_3$ | H | H | H | H | H | |
| 3.55 | $COCH_3$ | H | $CH_3$ | $CH_3$ | $CH_3$ | H | H | H | H | |
| 3.56 | $COCH_3$ | H | $CH_3$ | $CH_3$ | H | H | H | 6-$CH_3$ | H | |
| 3.57 | $COCH_3$ | H | $CH_3$ | $CH_3$ | H | H | H | 6-$OCH_3$ | H | |
| 3.58 | $COCH_3$ | H | $CH_3$ | $CH_3$ | $CH_3$ | H | H | 6-$OCH_3$ | H | |
| 3.59 | $COCH_3$ | H | $C_2H_5$ | $C_2H_5$ | H | H | H | H | H | |
| 3.60 | $COCH_3$ | H | $C_2H_5$ | $C_2H_5$ | $CH_3$ | H | H | H | H | |
| 3.61 | $CO_2C_2H_5$ | H | $CH_3$ | H | H | H | H | H | H | |
| 3.62 | $CO_2C_2H_5$ | H | $CH_3$ | H | $CH_3$ | H | H | H | H | |
| 3.63 | $CO_2C_2H_5$ | H | $CH_3$ | $CH_3$ | H | H | H | H | H | |
| 3.64 | $CO_2C_2H_5$ | H | $CH_3$ | $CH_3$ | $CH_3$ | H | H | H | H | |
| 3.65 | $CO_2C_2H_5$ | H | $C_2H_5$ | $C_2H_5$ | $CH_3$ | H | H | H | H | |
| 3.66 | $CO_2C_2H_5$ | H | $C_2H_5$ | $C_2H_5$ | H | H | H | H | H | |
| 3.67 | $CONH_2$ | H | $CH_3$ | H | H | H | H | H | H | |
| 3.68 | $CONH_2$ | H | $CH_3$ | H | $CH_3$ | H | H | H | H | |
| 3.69 | $CONH_2$ | H | $CH_3$ | $CH_3$ | H | H | H | H | H | |
| 3.70 | $CONH_2$ | H | $CH_3$ | $CH_3$ | $CH_3$ | H | H | H | H | |
| 3.71 | $CONH_2$ | H | $C_2H_5$ | $C_2H_5$ | H | H | H | H | H | |
| 3.72 | $CONH_2$ | H | $C_2H_5$ | $C_2H_5$ | $CH_3$ | H | H | H | H | |
| 3.73 | $CONHCH_3$ | H | $CH_3$ | H | H | H | H | H | H | |
| 3.74 | $CONHCH_3$ | H | $CH_3$ | H | $CH_3$ | H | H | H | H | |
| 3.75 | $CONHCH_3$ | H | $CH_3$ | $CH_3$ | H | H | H | H | H | |
| 3.76 | $CONHCH_3$ | H | $CH_3$ | $CH_3$ | $CH_3$ | H | H | H | H | |
| 3.77 | $CONHCH_3$ | H | $C_2H_5$ | $C_2H_5$ | H | H | H | H | H | |
| 3.78 | $CONHCH_3$ | H | $C_2H_5$ | $C_2H_5$ | $CH_3$ | H | H | H | H | |
| 3.79 | $CONHCH_3$ | H | $-CH_2-CH_2-$ | | H | H | H | H | H | |
| 3.80 | $CONHCH_3$ | H | $-CH_2-CH_2-$ | | $CH_3$ | H | H | H | H | |
| 3.81 | $CON(CH_3)_2$ | H | $CH_3$ | $CH_3$ | H | H | H | H | H | |
| 3.82 | $CON(CH_3)_2$ | H | $CH_3$ | $CH_3$ | $CH_3$ | H | H | H | H | |
| 3.83 | $CON(CH_3)_2$ | H | $C_2H_5$ | $C_2H_5$ | H | H | H | H | H | |
| 3.84 | $CON(CH_3)_2$ | H | $C_2H_5$ | $C_2H_5$ | $CH_3$ | H | H | H | H | |
| 3.85 | CN | H | $CH_3$ | $CH_3$ | H | H | H | H | H | |
| 3.86 | CN | H | $CH_3$ | $CH_3$ | $CH_3$ | H | H | H | H | |

Tabelle 4:

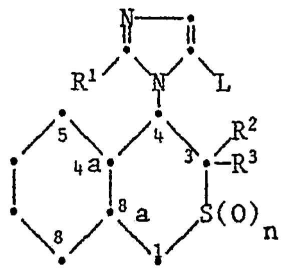

| Verb. Nr. | L | $R^1$ | $R^2$ | $R^3$ | n | Pos. 4a-8a | Pos. 4-4a | phys. Daten |
|---|---|---|---|---|---|---|---|---|
| 4.01 | $CO_2CH_3$ | H | H | H | 0 | - | - | |
| 4.02 | $CO_2CH_3$ | H | H | H | 0 | trans | trans | |
| 4.03 | $CO_2CH_3$ | H | H | H | 0 | trans | cis | Smp.157-158°C |
| 4.04 | $CO_2CH_3$ | SH | H | H | 0 | - | - | |
| 4.05 | $CO_2CH_3$ | H | $CH_3$ | H | 0 | - | - | |
| 4.06 | $CO_2CH_3$ | H | $CH_3$ | H | 0 | trans | trans | |
| 4.07 | $CO_2CH_3$ | H | $CH_3$ | H | 0 | trans | trans | |
| 4.08 | $CO_2CH_3$ | SH | $CH_3$ | H | 0 | - | - | |
| 4.09 | $CO_2CH_3$ | H | $CH_3$ | $CH_3$ | 0 | - | - | |
| 4.10 | $CO_2CH_3$ | H | $CH_3$ | $CH_3$ | 0 | trans | trans | |
| 4.11 | $CO_2CH_3$ | H | $CH_3$ | $CH_3$ | 0 | trans | trans | |
| 4.12 | $CO_2CH_3$ | H | $C_2H_5$ | H | 0 | - | - | |
| 4.13 | $CO_2CH_3$ | H | $C_2H_5$ | $C_2H_5$ | 0 | - | - | |
| 4.14 | $CO_2CH_3$ | H | $C_2H_5$ | $C_2H_5$ | 0 | trans | cis | |
| 4.15 | $CO_2CH_3$ | H | $C_2H_5$ | $C_2H_5$ | 0 | trans | trans | |
| 4.16 | $CO_2CH_3$ | H | $-CH_2-CH_2-$ | | 0 | - | - | |
| 4.17 | $CO_2CH_3$ | H | $C_2H_5$ | $CH_3$ | 0 | - | - | |
| 4.18 | $CO_2C_2H_5$ | H | H | H | 0 | - | - | |
| 4.19 | $CO_2C_2H_5$ | H | $CH_3$ | H | 0 | - | - | |
| 4.20 | $CO_2C_2H_5$ | H | $CH_3$ | $CH_3$ | 0 | - | - | |
| 4.21 | $CO_2C_2H_5$ | H | $CH_3$ | $CH_3$ | 0 | trans | - | |
| 4.22 | $CO_2C_2H_5$ | H | $C_2H_5$ | H | 0 | - | - | |
| 4.23 | $CO_2C_2H_5$ | H | $C_2H_5$ | $C_2H_5$ | 0 | - | - | |
| 4.24 | $CO_2C_2H_5$ | H | $C_2H_5$ | $C_2H_5$ | 0 | trans | - | |
| 4.25 | $CO_2C_2H_5$ | H | $-CH_2-CH_2-$ | | 0 | - | - | |
| 4.26 | $CO_2C_2H_5$ | H | $C_2H_5$ | $CH_3$ | 0 | - | - | |
| 4.27 | $CONH_2$ | H | $CH_3$ | H | 0 | - | - | |
| 4.28 | $CONH_2$ | H | $CH_3$ | $CH_3$ | 0 | - | - | |
| 4.29 | $CONH_2$ | H | $C_2H_5$ | H | 0 | - | - | |
| 4.30 | $CONH_2$ | H | $C_2H_5$ | $C_2H_5$ | 0 | - | - | |
| 4.31 | $CONHCH_3$ | H | H | H | 0 | - | - | |
| 4.32 | $CONHCH_3$ | H | $CH_3$ | H | 0 | - | - | |

Fortsetzung Tabelle 4:

| Verb. Nr. | L | $R^1$ | $R^2$ | $R^3$ | n | Pos. 4a-8a | Pos. 4-4a | phys. Daten |
|---|---|---|---|---|---|---|---|---|
| 4.33 | $CONHCH_3$ | H | $CH_3$ | $CH_3$ | 0 | - | - | |
| 4.34 | $CONHCH_3$ | H | $C_2H_5$ | H | 0 | - | - | |
| 4.35 | $CONHCH_3$ | H | $C_2H_5$ | $C_2H_5$ | 0 | - | - | |
| 4.36 | $CON(CH_3)_2$ | H | H | H | 0 | - | - | |
| 4.37 | $CON(CH_3)_2$ | H | $CH_3$ | H | 0 | - | - | |
| 4.38 | $CON(CH_3)_2$ | H | $CH_3$ | $CH_3$ | 0 | - | - | |
| 4.39 | $CON(CH_3)_2$ | H | $C_2H_5$ | $C_2H_5$ | 0 | - | - | |
| 4.40 | $COCH_3$ | H | H | H | 0 | - | - | |
| 4.41 | $COCH_3$ | H | $CH_3$ | $CH_3$ | 0 | - | - | |
| 4.42 | $COCH_3$ | H | $C_2H_5$ | $C_2H_5$ | 0 | - | - | |
| 4.43 | $COC_2H_5$ | H | H | H | 0 | - | - | |
| 4.44 | $COC_2H_5$ | H | $CH_3$ | $CH_3$ | 0 | - | - | |
| 4.45 | $CO_2CH_2CH=CH_2$ | H | H | H | 0 | - | - | |
| 4.46 | $CO_2CH_2C{\equiv}CH$ | H | H | H | 0 | - | - | |
| 4.47 | $CO_2CH_3$ | H | H | H | 1 | - | - | |
| 4.48 | $CO_2CH_3$ | H | $CH_3$ | H | 1 | - | - | |
| 4.49 | $CO_2CH_3$ | H | $CH_3$ | $CH_3$ | 1 | - | - | |
| 4.50 | $CO_2CH_3$ | H | $C_2H_5$ | $C_2H_5$ | 1 | - | - | |
| 4.51 | $CO_2C_2H_5$ | H | H | H | 1 | - | - | |
| 4.52 | $CO_2C_2H_5$ | H | $CH_3$ | $CH_3$ | 1 | - | - | |
| 4.53 | $CO_2C_2H_5$ | H | $C_2H_5$ | $C_2H_5$ | 1 | - | - | |
| 4.54 | $COCH_3$ | H | $CH_3$ | H | 1 | - | - | |
| 4.55 | $COCH_3$ | H | $CH_3$ | $CH_3$ | 1 | - | - | |
| 4.56 | $COCH_3$ | H | $C_2H_5$ | $C_2H_5$ | 1 | - | - | |
| 4.57 | $CO_2C_2H_5$ | H | $CH_3$ | $CH_3$ | 1 | - | - | |
| 4.58 | $CONH_2$ | H | $CH_3$ | $CH_3$ | 1 | - | - | |
| 4.59 | $CONHCH_3$ | H | $CH_3$ | $CH_3$ | 1 | - | - | |
| 4.60 | $CON(CH_3)_2$ | H | $CH_3$ | $CH_3$ | 1 | - | - | |
| 4.61 | CN | H | $CH_3$ | $CH_3$ | 1 | - | - | |
| 4.62 | $CO_2CH_3$ | H | H | H | 2 | - | - | |
| 4.63 | $CO_2CH_3$ | H | $CH_3$ | $CH_3$ | 2 | - | - | |
| 4.64 | $CO_2C_2H_5$ | H | $CH_3$ | $CH_3$ | 2 | - | - | |
| 4.65 | $COCH_3$ | H | $CH_3$ | $CH_3$ | 2 | - | - | |
| 4.66 | $COC_2H_5$ | H | $CH_3$ | $CH_3$ | 2 | - | - | |
| 4.67 | $CONH_2$ | H | $CH_3$ | $CH_3$ | 2 | - | - | |
| 4.68 | $CONHCH_3$ | H | $CH_3$ | $CH_3$ | 2 | - | - | |
| 4.69 | $CON(CH_3)_2$ | H | $CH_3$ | $CH_3$ | 2 | - | - | |
| 4.70 | CN | H | $CH_3$ | $CH_3$ | 2 | - | - | |

Tabelle 5:

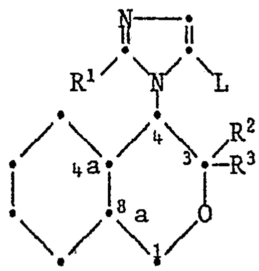

| Verb. Nr. | L | $R^1$ | $R^2$ | $R^3$ | Pos. 4a-8a | Pos. 4-4a | phys. Daten |
|---|---|---|---|---|---|---|---|
| 5.01 | $CO_2CH_3$ | H | H | H | - | - | |
| 5.02 | $CO_2CH_3$ | H | H | H | trans | trans | |
| 5.03 | $CO_2CH_3$ | H | H | H | trans | cis | |
| 5.04 | $CO_2CH_3$ | SH | H | H | - | - | |
| 5.05 | $CO_2CH_3$ | H | $CH_3$ | H | - | - | |
| 5.06 | $CO_2CH_3$ | H | $CH_3$ | H | trans | trans | |
| 5.07 | $CO_2CH_3$ | H | $CH_3$ | H | trans | trans | |
| 5.08 | $CO_2CH_3$ | SH | $CH_3$ | H | - | - | |
| 5.09 | $CO_2CH_3$ | H | $CH_3$ | $CH_3$ | - | - | |
| 5.10 | $CO_2CH_3$ | H | $CH_3$ | $CH_3$ | trans | trans | |
| 5.11 | $CO_2CH_3$ | H | $CH_3$ | $CH_3$ | trans | trans | |
| 5.12 | $CO_2CH_3$ | H | $C_2H_5$ | H | - | - | |
| 5.13 | $CO_2CH_3$ | H | $C_2H_5$ | $C_2H_5$ | - | - | |
| 5.14 | $CO_2CH_3$ | H | $C_2H_5$ | $C_2H_5$ | trans | cis | |
| 5.15 | $CO_2CH_3$ | H | $C_2H_5$ | $C_2H_5$ | trans | trans | |
| 5.16 | $CO_2CH_3$ | H | $-CH_2-CH_2-$ | | - | - | |
| 5.17 | $CO_2CH_3$ | H | $C_2H_5$ | $CH_3$ | - | - | |
| 5.18 | $CO_2C_2H_5$ | H | H | H | - | - | |
| 5.19 | $CO_2C_2H_5$ | H | $CH_3$ | H | - | - | |
| 5.20 | $CO_2C_2H_5$ | H | $CH_3$ | $CH_3$ | - | - | |
| 5.21 | $CO_2C_2H_5$ | H | $CH_3$ | $CH_3$ | trans | - | |
| 5.22 | $CO_2C_2H_5$ | H | $C_2H_5$ | H | - | - | |
| 5.23 | $CO_2C_2H_5$ | H | $C_2H_5$ | $C_2H_5$ | - | - | |
| 5.24 | $CO_2C_2H_5$ | H | $C_2H_5$ | $C_2H_5$ | trans | - | |
| 5.25 | $CO_2C_2H_5$ | H | $-CH_2-CH_2-$ | | - | - | |
| 5.26 | $CO_2C_2H_5$ | H | $C_2H_5$ | $CH_3$ | - | - | |
| 5.27 | $CONH_2$ | H | $CH_3$ | H | - | - | |
| 5.28 | $CONH_2$ | H | $CH_3$ | $CH_3$ | - | - | |
| 5.29 | $CONH_2$ | H | $C_2H_5$ | H | - | - | |
| 5.30 | $CONH_2$ | H | $C_2H_5$ | $C_2H_5$ | - | - | |
| 5.31 | $CONHCH_3$ | H | H | H | - | - | |
| 5.32 | $CONHCH_3$ | H | $CH_3$ | H | - | - | |

Fortsetzung Tabelle 5:

| Verb. Nr. | L | $R^1$ | $R^2$ | $R^3$ | Pos. 4a-8a | Pos. 4-4a | phys. Daten |
|---|---|---|---|---|---|---|---|
| 5.33 | $CONHCH_3$ | H | $CH_3$ | $CH_3$ | - | - | |
| 5.34 | $CONHCH_3$ | H | $C_2H_5$ | H | - | - | |
| 5.35 | $CONHCH_3$ | H | $C_2H_5$ | $C_2H_5$ | - | - | |
| 5.36 | $CON(CH_3)_2$ | H | H | H | - | - | |
| 5.37 | $CON(CH_3)_2$ | H | $CH_3$ | H | - | - | |
| 5.38 | $CON(CH_3)_2$ | H | $CH_3$ | $CH_3$ | - | - | |
| 5.39 | $CON(CH_3)_2$ | H | $C_2H_5$ | $C_2H_5$ | - | - | |
| 5.40 | $COCH_3$ | H | H | H | - | - | |
| 5.41 | $COCH_3$ | H | $CH_3$ | $CH_3$ | - | - | |
| 5.42 | $COCH_3$ | H | $C_2H_5$ | $C_2H_5$ | - | - | |
| 5.43 | $COC_2H_5$ | H | H | H | - | - | |
| 5.44 | $COC_2H_5$ | H | $CH_3$ | $CH_3$ | - | - | |
| 5.45 | $CO_2CH_2CH{=}CH_2$ | H | H | H | - | - | |
| 5.46 | $CO_2CH_2C{\equiv}CH$ | H | H | H | - | - | |

Tabelle 6:

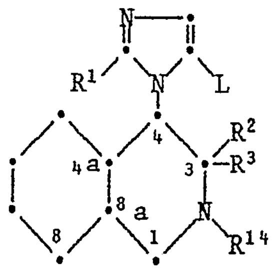

| Verb. Nr. | L | $R^1$ | $R^2$ | $R^3$ | $R^{14}$ | phys. Daten |
|---|---|---|---|---|---|---|
| 6.01 | $CO_2CH_3$ | H | H | H | H | |
| 6.02 | $CO_2CH_3$ | H | H | H | $CH_3$ | |
| 6.03 | $CO_2CH_3$ | H | H | H | Benzyl | |
| 6.04 | $CO_2CH_3$ | H | $CH_3$ | H | H | |
| 6.05 | $CO_2CH_3$ | H | $CH_3$ | H | $CH_3$ | |
| 6.06 | $CO_2CH_3$ | H | $CH_3$ | $CH_3$ | H | |
| 6.07 | $CO_2CH_3$ | H | $CH_3$ | $CH_3$ | $CH_3$ | |
| 6.08 | $CO_2CH_3$ | H | $C_2H_5$ | H | H | |
| 6.09 | $CO_2CH_3$ | H | $C_2H_5$ | H | $CH_3$ | |
| 6.10 | $CO_2CH_3$ | H | $C_2H_5$ | $C_2H_5$ | H | |
| 6.11 | $CO_2CH_3$ | H | $C_2H_5$ | $C_2H_5$ | $CH_3$ | |
| 6.12 | $CO_2CH_3$ | H | $C_3H_7$-i | H | H | |
| 6.13 | $CO_2CH_3$ | H | $C_3H_7$-i | H | $CH_3$ | |
| 6.14 | $CO_2CH_3$ | H | $C_4H_9$-i | H | H | |
| 6.15 | $CO_2CH_3$ | H | $C_4H_9$-i | H | $CH_3$ | |
| 6.16 | $CO_2C_2H_5$ | H | $CH_3$ | H | H | |
| 6.17 | $CO_2C_2H_5$ | H | $CH_3$ | $CH_3$ | H | |
| 6.18 | $CO_2C_2H_5$ | H | $CH_3$ | $CH_3$ | $CH_3$ | |
| 6.19 | $CO_2C_2H_5$ | H | $C_2H_5$ | H | $CH_3$ | |
| 6.20 | $CO_2C_2H_5$ | H | $C_2H_5$ | $C_2H_5$ | $CH_3$ | |
| 6.21 | $CO_2C_2H_5$ | H | $C_3H_7$-i | H | $CH_3$ | |
| 6.22 | $COCH_3$ | H | $CH_3$ | $CH_3$ | H | |
| 6.23 | $COCH_3$ | H | $CH_3$ | $CH_3$ | $CH_3$ | |
| 6.24 | $COCH_3$ | H | $C_2H_5$ | H | $CH_3$ | |
| 6.25 | $COCH_3$ | H | $C_2H_5$ | $C_2H_5$ | $CH_3$ | |
| 6.26 | $COCH_3$ | H | $C_2H_5$ | $C_2H_5$ | H | |
| 6.27 | $COC_2H_5$ | H | $CH_3$ | $CH_3$ | H | |
| 6.28 | $COC_2H_5$ | H | $CH_3$ | $CH_3$ | $CH_3$ | |
| 6.29 | $COC_2H_5$ | H | $C_2H_5$ | H | $CH_3$ | |
| 6.30 | $COC_2H_5$ | H | $C_2H_5$ | $C_2H_5$ | $CH_3$ | |
| 6.31 | $COC_2H_5$ | H | $C_2H_5$ | $C_2H_5$ | H | |
| 6.32 | $CONH_2$ | H | $CH_3$ | $CH_3$ | H | |

Fortsetzung Tabelle 6:

| Verb. Nr. | L | $R^1$ | $R^2$ | $R^3$ | $R^{14}$ | phys. Daten |
|---|---|---|---|---|---|---|
| 6.33 | $CONH_2$ | H | $CH_3$ | $CH_3$ | $CH_3$ | |
| 6.34 | $CONH_2$ | H | $C_2H_5$ | $C_2H_5$ | H | |
| 6.35 | $CONHCH_3$ | H | $CH_3$ | $CH_3$ | H | |
| 6.36 | $CON(CH_3)_2$ | H | $CH_3$ | $CH_3$ | $CH_3$ | |
| 6.37 | $CON(CH_3)_2$ | H | $CH_3$ | $CH_3$ | H | |
| 6.38 | $CON(CH_3)_2$ | H | $CH_3$ | $CH_3$ | $CH_3$ | |
| 6.39 | $CO_2CH_2CH=CH_2$ | H | $CH_3$ | $CH_3$ | H | |
| 6.40 | $CO_2CH_2CH=CH_2$ | H | $CH_3$ | $CH_3$ | $CH_3$ | |
| 6.41 | $CO_2CH_2C\equiv CH$ | H | $CH_3$ | $CH_3$ | H | |
| 6.42 | $CO_2CH_2C\equiv CH$ | H | $CH_3$ | $CH_3$ | $CH_3$ | |
| 6.43 | CN | H | $CH_3$ | $CH_3$ | H | |
| 6.44 | CN | H | $CH_3$ | $CH_3$ | $CH_3$ | |

Tabelle 7:

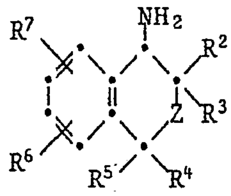

| Verb. Nr. | $R^2$ | $R^3$ | Z | $R^4$ | $R^5$ | $R^6$ | $R^7$ | phys. Daten |
|---|---|---|---|---|---|---|---|---|
| 7.01 | H | H | S | H | H | H | H | $Sdp._{0,06}$= 80°C |
| 7.02 | H | H | S | $CH_3$ | H | H | H | |
| 7.03 | $CH_3$ | H | S | H | H | H | H | |
| 7.04 | $CH_3$ | $CH_3$ | S | H | H | H | H | $Sdp._{0,06}$= 80°C |
| 7.05 | $CH_3$ | $CH_3$ | S | $CH_3$ | H | H | H | |
| 7.06 | $CH_3$ | $CH_3$ | S | H | H | 7-$OCH_3$ | H | |
| 7.07 | $CH_3$ | $CH_3$ | S | $CH_3$ | H | 6-$CH_3$ | H | |
| 7.08 | $CH_3$ | $CH_3$ | S | H | H | 5-$CH_3$ | 8-$CH_3$ | |
| 7.09 | $-CH_2-CH_2-$ | | S | H | H | H | H | |
| 7.10 | H | H | S | $C_2H_5$ | H | H | H | |
| 7.11 | $C_2H_5$ | H | S | H | H | H | H | $Sdp._{0,04}$= 90°C |
| 7.12 | $C_2H_5$ | $C_2H_5$ | S | H | H | H | H | |
| 7.13 | $C_2H_5$ | $C_2H_5$ | S | $CH_3$ | H | H | H | |
| 7.14 | $C_2H_5$ | $C_2H_5$ | S | H | H | 6-$CH_3$ | H | |
| 7.15 | H | H | O | H | H | H | H | |
| 7.16 | H | H | O | $CH_3$ | H | H | H | |
| 7.17 | $CH_3$ | H | O | H | H | H | H | |
| 7.18 | $CH_3$ | $CH_3$ | O | H | H | H | H | |
| 7.19 | $CH_3$ | $CH_3$ | O | $CH_3$ | H | H | H | |
| 7.20 | $CH_3$ | $CH_3$ | O | H | H | 7-$OCH_3$ | H | |
| 7.21 | $CH_3$ | $CH_3$ | O | $CH_3$ | H | 6-$CH_3$ | H | |
| 7.22 | $CH_3$ | $CH_3$ | O | H | H | 5-$CH_3$ | 8-$CH_3$ | |
| 7.23 | $-CH_2-CH_2-$ | | O | H | H | H | H | |
| 7.24 | H | H | O | $C_2H_5$ | H | H | H | |
| 7.25 | $C_2H_5$ | H | O | H | H | H | H | |
| 7.26 | $C_2H_5$ | $C_2H_5$ | O | H | H | H | H | |
| 7.27 | $C_2H_5$ | $C_2H_5$ | O | $CH_3$ | H | H | H | |
| 7.28 | $C_2H_5$ | $C_2H_5$ | O | H | H | 6-$CH_3$ | H | |
| 7.29 | $CH_3$ | H | $NCH_3$ | H | H | H | H | |
| 7.30 | $CH_3$ | H | $NCH_3$ | $CH_3$ | H | H | H | |
| 7.31 | $CH_3$ | $CH_3$ | $NCH_3$ | H | H | H | H | |
| 7.32 | $CH_3$ | $CH_3$ | NH | H | H | H | H | |
| 7.33 | $CH_3$ | $CH_3$ | NH | H | H | 6-$OCH_3$ | 7-$OCH_3$ | |
| 7.34 | $C_2H_5$ | H | $NCH_3$ | H | H | H | H | |
| 7.35 | $C_2H_5$ | $C_2H_5$ | NH | H | H | H | H | |
| 7.36 | $C_2H_5$ | $C_2H_5$ | $NCH_3$ | H | H | H | H | |

Fortsetzung Tabelle 7:

| Verb. Nr. | $R^2$ | $R^3$ | Z | $R^4$ | $R^5$ | $R^6$ | $R^7$ | phys. Daten |
|---|---|---|---|---|---|---|---|---|
| 7.37 | H | H | SO | H | H | H | H | |
| 7.38 | H | H | SO | $CH_3$ | H | H | H | |
| 7.39 | $CH_3$ | H | SO | H | H | H | H | |
| 7.40 | $CH_3$ | $CH_3$ | SO | H | H | H | H | |
| 7.41 | $CH_3$ | $CH_3$ | SO | $CH_3$ | H | H | H | |
| 7.42 | $CH_3$ | $CH_3$ | SO | H | H | 7-$OCH_3$ | H | |
| 7.43 | $CH_3$ | $CH_3$ | SO | $CH_3$ | H | 6-$CH_3$ | H | |
| 7.44 | $CH_3$ | $CH_3$ | SO | H | H | 5-$CH_3$ | 8-$CH_3$ | |
| 7.45 | $-CH_2-CH_2-$ | | SO | H | H | H | H | |
| 7.46 | H | H | SO | $C_2H_5$ | H | H | H | |
| 7.47 | $C_2H_5$ | H | SO | H | H | H | H | |
| 7.48 | $C_2H_5$ | $C_2H_5$ | SO | H | H | H | H | |
| 7.49 | $C_2H_5$ | $C_2H_5$ | SO | $CH_3$ | H | H | H | |
| 7.50 | $C_2H_5$ | $C_2H_5$ | $SO_2$ | H | H | 6-$CH_3$ | H | |
| 7.51 | H | H | $SO_2$ | H | H | H | H | |
| 7.52 | H | H | $SO_2$ | $CH_3$ | H | H | H | |
| 7.53 | $CH_3$ | H | $SO_2$ | H | H | H | H | |
| 7.54 | $CH_3$ | $CH_3$ | $SO_2$ | H | H | H | H | |
| 7.55 | $CH_3$ | $CH_3$ | $SO_2$ | $CH_3$ | H | H | H | |
| 7.56 | $CH_3$ | $CH_3$ | $SO_2$ | H | H | 7-$OCH_3$ | H | |
| 7.57 | $CH_3$ | $CH_3$ | $SO_2$ | $CH_3$ | H | 6-$CH_3$ | H | |
| 7.58 | $CH_3$ | $CH_3$ | $SO_2$ | H | H | 5-$CH_3$ | 8-$CH_3$ | |
| 7.59 | $-CH_2-CH_2-$ | | $SO_2$ | H | H | H | H | |
| 7.60 | H | H | $SO_2$ | $C_2H_5$ | H | H | H | |
| 7.61 | $C_2H_5$ | H | $SO_2$ | H | H | H | H | |
| 7.62 | $C_2H_5$ | $C_2H_5$ | $SO_2$ | H | H | H | H | |
| 7.63 | $C_2H_5$ | $C_2H_5$ | $SO_2$ | $CH_3$ | H | H | H | |
| 7.64 | $C_2H_5$ | $C_2H_5$ | $SO_2$ | H | H | 6-$CH_3$ | H | |

Tabelle 8:

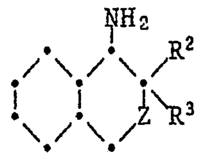

| Verb. Nr. | $R^2$ | $R^3$ | Z | phys. Daten |
|---|---|---|---|---|
| 8.01 | H | H | S | Sdp.$_{0,02}$= 60°C |
| 8.02 | $CH_3$ | H | S | |
| 8.03 | $CH_3$ | $CH_3$ | S | |
| 8.04 | $C_2H_5$ | H | S | |
| 8.05 | $C_2H_5$ | $C_2H_5$ | S | |
| 8.06 | $-CH_2-CH_2-$ | | S | |
| 8.07 | H | H | O | |
| 8.08 | $CH_3$ | H | O | |
| 8.09 | $CH_3$ | $CH_3$ | O | |
| 8.10 | $C_2H_5$ | H | O | |
| 8.11 | $C_2H_5$ | $C_2H_5$ | O | |
| 8.12 | $-CH_2-CH_2-$ | | O | |
| 8.13 | H | H | NH | |
| 8.14 | H | H | $NCH_3$ | |
| 8.15 | $CH_3$ | H | $NCH_3$ | |
| 8.16 | $CH_3$ | $CH_3$ | NH | |
| 8.17 | $CH_3$ | $CH_3$ | $NCH_3$ | |
| 8.18 | $C_2H_5$ | H | NH | |
| 8.19 | $C_2H_5$ | H | $NCH_3$ | |
| 8.20 | $C_2H_5$ | $C_2H_5$ | NH | |
| 8.21 | $C_2H_5$ | $C_2H_5$ | $NCH_3$ | |

Tabelle 9:

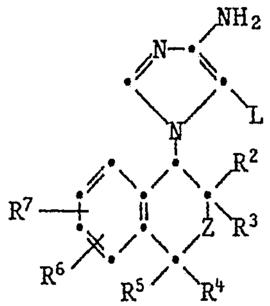

| Verb. Nr. | L | $R^2$ | $R^3$ | Z | $R^4$ | $R^5$ | $R^6$ | $R^7$ | phys. Daten |
|---|---|---|---|---|---|---|---|---|---|
| 9.01 | $CO_2CH_3$ | H | H | S | H | H | H | H | |
| 9.02 | $CO_2CH_3$ | $CH_3$ | H | S | H | H | H | H | |
| 9.03 | $CO_2CH_3$ | $CH_3$ | H | S | $CH_3$ | H | H | H | |
| 9.04 | $CO_2CH_3$ | $CH_3$ | H | S | $CH_3$ | H | 5-$CH_3$ | H | |
| 9.05 | $CO_2CH_3$ | $CH_3$ | H | S | $CH_3$ | H | 6-$CH_3$ | H | |
| 9.06 | $CO_2CH_3$ | $CH_3$ | H | S | $CH_3$ | H | 6-$OCH_3$ | H | |
| 9.07 | $CO_2CH_3$ | $CH_3$ | H | S | $CH_3$ | H | 5-$CH_3$ | 8-$CH_3$ | |
| 9.08 | $CO_2CH_3$ | $CH_3$ | $CH_3$ | S | H | H | H | H | |
| 9.09 | $CO_2CH_3$ | $CH_3$ | $CH_3$ | S | $CH_3$ | H | H | H | |
| 9.10 | $CO_2CH_3$ | $CH_3$ | $CH_3$ | S | H | H | 6-$CH_3$ | H | |
| 9.11 | $CO_2CH_3$ | $CH_3$ | $CH_3$ | S | H | H | 6-$OCH_3$ | H | |
| 9.12 | $CO_2CH_3$ | $CH_3$ | $CH_3$ | S | H | H | 6-$NO_2$ | H | |
| 9.13 | $CO_2CH_3$ | $CH_3$ | $CH_3$ | S | H | H | 5-F | H | |
| 9.14 | $CO_2CH_3$ | $-CH_2-CH_2-$ | | S | H | H | H | H | |
| 9.15 | $CO_2CH_3$ | $C_2H_5$ | H | S | H | H | H | H | Smp. 149-150°C |
| 9.16 | $CO_2CH_3$ | $C_2H_5$ | $C_2H_5$ | S | H | H | H | H | |
| 9.17 | $CO_2CH_3$ | $C_2H_5$ | $C_2H_5$ | S | H | H | 6-$CH_3$ | H | |
| 9.18 | $CO_2CH_3$ | $C_2H_5$ | $C_2H_5$ | S | $CH_3$ | H | H | H | |
| 9.19 | $CO_2CH_3$ | $-(CH_2)_4-$ | | S | H | H | H | H | |
| 9.20 | $CO_2C_2H_5$ | H | H | S | H | H | H | H | |
| 9.21 | $CO_2C_2H_5$ | $CH_3$ | H | S | H | H | H | H | |
| 9.22 | $CO_2C_2H_5$ | $CH_3$ | $CH_3$ | S | H | H | H | H | |
| 9.23 | $CO_2C_2H_5$ | $CH_3$ | $CH_3$ | S | H | H | 6-$OCH_3$ | H | |
| 9.24 | $CO_2C_2H_5$ | $C_2H_5$ | $C_2H_5$ | S | H | H | H | H | |
| 9.25 | $CO_2C_2H_5$ | $C_2H_5$ | H | S | H | H | H | H | |
| 9.26 | $CO_2C_2H_5$ | $-CH_2-CH_2-$ | | S | H | H | H | H | |
| 9.27 | $COCH_3$ | $CH_3$ | H | S | H | H | H | H | |
| 9.28 | $COCH_3$ | $CH_3$ | $CH_3$ | S | H | H | H | H | |
| 9.29 | $COC_2H_5$ | $CH_3$ | $CH_3$ | S | H | H | H | H | |
| 9.30 | $CON(CH_3)_2$ | $CH_3$ | $CH_3$ | S | H | H | H | H | |
| 9.31 | $CO_2CH_3$ | $CH_3$ | H | SO | H | H | H | H | |
| 9.32 | $CO_2CH_3$ | $CH_3$ | $CH_3$ | SO | H | H | H | H | |
| 9.33 | $CO_2CH_3$ | $C_2H_5$ | H | SO | H | H | H | H | |
| 9.34 | $CO_2CH_3$ | $C_2H_5$ | $C_2H_5$ | SO | H | H | H | H | |
| 9.35 | $CO_2C_2H_5$ | $CH_3$ | H | SO | H | H | H | H | |
| 9.36 | $CO_2C_2H_5$ | $CH_3$ | $CH_3$ | SO | H | H | H | H | |

Fortsetzung Tabelle 9:

| Verb. Nr. | L | $R^2$ | $R^3$ | Z | $R^4$ | $R^5$ | $R^6$ | $R^7$ | phys. Daten |
|---|---|---|---|---|---|---|---|---|---|
| 9.37 | $CO_2C_2H_5$ | $C_2H_5$ | $C_2H_5$ | SO | H | H | H | H | |
| 9.38 | $COCH_3$ | $CH_3$ | $CH_3$ | SO | H | H | H | H | |
| 9.39 | $CO_2C_2H_5$ | $CH_3$ | $CH_3$ | SO | H | H | H | H | |
| 9.40 | $CON(CH_3)_2$ | $CH_3$ | $CH_3$ | SO | H | H | H | H | |
| 9.41 | $CO_2CH_3$ | $CH_3$ | H | $SO_2$ | H | H | H | H | |
| 9.42 | $CO_2CH_3$ | $CH_3$ | $CH_3$ | $SO_2$ | H | H | H | H | |
| 9.43 | $CO_2CH_3$ | $C_2H_5$ | H | $SO_2$ | H | H | H | H | |
| 9.44 | $CO_2CH_3$ | $C_2H_5$ | $C_2H_5$ | $SO_2$ | H | H | H | H | |
| 9.45 | $CO_2CH_3$ | $-CH_2-CH_2-$ | | $SO_2$ | H | H | H | H | |
| 9.46 | $CO_2C_2H_5$ | $CH_3$ | H | $SO_2$ | H | H | H | H | |
| 9.47 | $CO_2C_2H_5$ | $CH_3$ | $CH_3$ | $SO_2$ | H | H | H | H | |
| 9.48 | $CO_2C_2H_5$ | $C_2H_5$ | $C_2H_5$ | $SO_2$ | H | H | H | H | |
| 9.49 | $COCH_3$ | $CH_3$ | $CH_3$ | $SO_2$ | H | H | H | H | |
| 9.50 | $CO_2C_2H_5$ | $CH_3$ | $CH_3$ | $SO_2$ | H | H | H | H | |
| 9.51 | $CON(CH_3)_2$ | $CH_3$ | $CH_3$ | $SO_2$ | H | H | H | H | |
| 9.52 | $CO_2CH_3$ | H | H | O | H | H | H | H | |
| 9.53 | $CO_2CH_3$ | $CH_3$ | H | O | H | H | H | H | |
| 9.54 | $CO_2CH_3$ | $CH_3$ | $CH_3$ | O | H | H | H | H | |
| 9.55 | $CO_2CH_3$ | $C_2H_5$ | H | O | H | H | H | H | |
| 9.56 | $CO_2CH_3$ | $C_2H_5$ | $C_2H_5$ | O | H | H | H | H | |
| 9.57 | $CO_2CH_3$ | $-CH_2-CH_2-$ | | O | H | H | H | H | |
| 9.58 | $CO_2C_2H_5$ | $CH_3$ | H | O | H | H | H | H | |
| 9.59 | $CO_2C_2H_5$ | $CH_3$ | $CH_3$ | O | H | H | H | H | |
| 9.60 | $CO_2C_2H_5$ | $C_2H_5$ | $C_2H_5$ | O | H | H | H | H | |
| 9.61 | $COCH_3$ | $CH_3$ | H | O | H | H | H | H | |
| 9.62 | $COCH_3$ | $CH_3$ | $CH_3$ | O | H | H | H | H | |
| 9.63 | $COCH_3$ | $C_2H_5$ | H | O | H | H | H | H | |
| 9.64 | $COCH_3$ | $C_2H_5$ | $C_2H_5$ | O | H | H | H | H | |
| 9.65 | $CO_2C_2H_5$ | $CH_3$ | $CH_3$ | O | H | H | H | H | |
| 9.66 | $CO_2C_2H_5$ | $C_2H_5$ | $C_2H_5$ | O | H | H | H | H | |
| 9.67 | $CON(CH_3)_2$ | $CH_3$ | $CH_3$ | O | H | H | H | H | |
| 9.68 | $CO_2CH_3$ | H | H | $NCH_3$ | H | H | H | H | |
| 9.69 | $CO_2CH_3$ | $CH_3$ | H | $NCH_3$ | H | H | H | H | |
| 9.70 | $CO_2CH_3$ | $CH_3$ | $CH_3$ | $NCH_3$ | H | H | H | H | |
| 9.71 | $CO_2CH_3$ | $C_2H_5$ | H | $NCH_3$ | H | H | H | H | |
| 9.72 | $CO_2CH_3$ | $C_2H_5$ | $C_2H_5$ | $NCH_3$ | H | H | H | H | |
| 9.73 | $CO_2C_2H_5$ | $CH_3$ | $CH_3$ | $NCH_3$ | H | H | H | H | |
| 9.74 | $CO_2C_2H_5$ | $C_2H_5$ | $C_2H_5$ | $NCH_3$ | H | H | H | H | |
| 9.75 | $COCH_3$ | $CH_3$ | H | $NCH_3$ | H | H | H | H | |
| 9.76 | $COCH_3$ | $CH_3$ | $CH_3$ | $NCH_3$ | H | H | H | H | |
| 9.77 | $COCH_3$ | $C_2H_5$ | H | $NCH_3$ | H | H | H | H | |
| 9.78 | $COCH_3$ | $C_2H_5$ | $C_2H_5$ | $NCH_3$ | H | H | H | H | |
| 9.79 | $COC_2H_5$ | $CH_3$ | H | $NCH_3$ | H | H | H | H | |
| 9.80 | $COC_2H_5$ | $CH_3$ | $CH_3$ | $NCH_3$ | H | H | H | H | |

Tabelle 10:

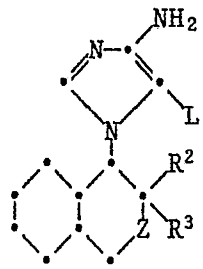

| Verb. Nr. | L | $R^2$ | $R^3$ | Z | phys. Daten |
|---|---|---|---|---|---|
| 10.01 | $CO_2CH_3$ | H | H | S | |
| 10.02 | $CO_2CH_3$ | $CH_3$ | H | S | |
| 10.03 | $CO_2CH_3$ | $CH_3$ | $CH_3$ | S | |
| 10.04 | $CO_2CH_3$ | $C_2H_5$ | H | S | |
| 10.05 | $CO_2CH_3$ | $C_2H_5$ | $C_2H_5$ | S | |
| 10.06 | $CO_2C_2H_5$ | $CH_3$ | $CH_3$ | S | |
| 10.07 | $CO_2C_2H_5$ | $C_2H_5$ | H | S | |
| 10.08 | $CO_2C_2H_5$ | $C_2H_5$ | $C_2H_5$ | S | |
| 10.09 | $COCH_3$ | $CH_3$ | $CH_3$ | S | |
| 10.10 | $CON(CH_3)_2$ | $CH_3$ | $CH_3$ | S | |
| 10.11 | $CO_2CH_3$ | H | H | SO | |
| 10.12 | $CO_2CH_3$ | $CH_3$ | $CH_3$ | SO | |
| 10.13 | $CO_2C_2H_5$ | $CH_3$ | $CH_3$ | SO | |
| 10.14 | $COCH_3$ | $CH_3$ | $CH_3$ | SO | |
| 10.15 | $CO_2CH_3$ | H | H | $SO_2$ | |
| 10.16 | $CO_2CH_3$ | $CH_3$ | $CH_3$ | $SO_2$ | |
| 10.17 | $CO_2C_2H_5$ | $CH_3$ | $CH_3$ | $SO_2$ | |
| 10.18 | $COCH_3$ | $CH_3$ | $CH_3$ | $SO_2$ | |
| 10.19 | $CO_2CH_3$ | H | H | O | |
| 10.20 | $CO_2CH_3$ | $CH_3$ | H | O | |
| 10.21 | $CO_2CH_3$ | $CH_3$ | $CH_3$ | O | |
| 10.22 | $CO_2C_2H_5$ | $C_2H_5$ | H | O | |
| 10.23 | $CO_2C_2H_5$ | $C_2H_5$ | $C_2H_5$ | O | |
| 10.24 | $COCH_3$ | $CH_3$ | $CH_3$ | O | |
| 10.25 | $CO_2CH_3$ | H | H | $NCH_3$ | |
| 10.26 | $CO_2CH_3$ | $CH_3$ | H | $NCH_3$ | |
| 10.27 | $CO_2CH_3$ | $CH_3$ | $CH_3$ | $NCH_3$ | |

Formulierungsbeispiele

Beispiel F1: Formulierungsbeispiele für flüssige Wirkstoffe der Formel I (% = Gewichtsprozent)

| a) Emulsionskonzentrate | a) | b) | c) |
|---|---|---|---|
| Wirkstoff der Formel I | 20 % | 40 % | 50 % |
| Ca-Dodecylbenzolsulfonat | 5 % | 8 % | 5,8 % |
| Ricinusöl-polyäthylenglykoläther (36 Mol AeO) | 5 % | - | - |
| Tributylphenol-polyäthylenglykoläther (30 mol AeO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | a) | b) | c) | d) |
|---|---|---|---|---|
| Wirkstoff der Formel I | 80 % | 10 % | 5 % | 95 % |
| Aethylenglykol-monomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°) | - | - | 94 % | - |

Die Lösungen sind zur Anwendung in form kleinster Tropfen geeignet.

| c) Granulate | a) | b) |
|---|---|---|
| Wirkstoff der Formel I | 5 % | 10 % |
| Kaolin | 94 % | - |
| Hochdisperse Kieselsäure | 1 % | |
| Attapulgit | - | 90 % |

Der Wirkstoff wird in Methylenchlorid gelöst, auf den Träger aufgesprüht und das Lösungsmittel anschliessend im Vakuum abgedampft.

| d) Stäubemittel | a) | b) |
|---|---|---|
| Wirkstoff der Formel I | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertiges Stäubemittel.

Beispiel F2: Formulierungsbeispiele für feste Wirkstoffe der Formel I (% = Gewichtsprozent)

| a) Spritzpulver | a) | b) |
|---|---|---|
| Wirkstoff Nr. 1.016 | 20 % | 60 % |
| Na-Ligninsulfonat | 5 % | 5 % |
| Na-Laurylsulfat | 3 % | - |
| Na-Diisobutylnaphthalinsulfonat | - | 6 % |
| Octylphenolpolyäthylenglykoläther (7-8 Mol AeO) | - | 2 % |
| Hochdisperse Kieselsäure | 5 % | 27 % |
| Kaolin | 67 % | - |

Der Wirkstoff wird mit den Zusatzstoffen gut vermischt und in einer geeigneten Mühle gut vermahlen. Man erhält Spritzpulver, die sich mit Wasser zur Suspension jeder gewünschten Konzentration verdünnen lassen.

b) Emulsions-Konzentrat

| | |
|---|---|
| Wirkstoff Nr. 1.016 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff Nr. 1.016 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

d) Extruder Granulat

Wirkstoff Nr. 2.034 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

Wirkstoff Nr. 2.034 3 %
Polyäthylenglykol (MG 200) 3 %
Kaolin 94 %

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

Wirkstoff Nr. 1.016 40 %
Aethylenglykol 10 %
Nonylphenolpolyäthylenglykoläther (15 Mol AeO) 6 %
Na-Ligninsulfonat 10 %
Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel B1: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25%igen Emulsionskonzentrat behandelt. Es werden Konzentrationen von 4 kg Wirksubstanz/Hektar angewendet. Die Saatschalen werden im Gewächshaus bei 22-25°C und 50-70 % relativer Luftfeuchtigkeit gehalten und der Versuch nach 3 Wochen nach der folgenden Notenskala ausgewertet.

1 : Pflanze nicht gekeimt oder total abgestorben
2-3: sehr starke Wirkung
4-6: mittlere Wirkung
7-8: schwache Wirkung
9: keine Wirkung (wie unbehandelte Kontrolle).

In diesem Versuch zeigen die Verbindungen der Tabellen 1 bis 6 starke Herbizidwirkung.

Testresultate: preemergente Prüfung

Aufwandmange: 4 kg Aktivsubstanz pro Hektar

| Verb. Nr. | Avena | Setaria | Sinapis | Stellaria |
|---|---|---|---|---|
| 1.016 | 7 | 3 | 1 | 2 |
| 2.034 | 2 | 1 | 2 | 2 |
| 3.03 | 1 | 3 | 1 | 2 |

Beispiel B2: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26°C und 45-60 % relativer Luftfeuchtigkeit gehalten. 15 Tage nach der Behandlung wird der Versuch nach der gleichen Notenskala wie im preemergenten Versuch ausgewertet.

Auch in diesem Versuch zeigen die Verbindungen der Tabellen 1 bis 6 gute Herbizidwirkung.

Testresultate: postemergente Prüfung

Aufwandmenge: 4 kg Aktivsubstanz pro Hektar

| Verb. Nr. | Avena | Setaria | Lolium | Solanum | Sinapis | Stellaria | Phaseolus |
|---|---|---|---|---|---|---|---|
| 1.016 | 5 | 7 | 5 | 3 | 3 | 4 | 3 |
| 2.034 | 4 | 3 | 4 | 4 | 3 | 6 | 2 |

Beispiel B3: Herbizidwirkung vor dem Auflaufen der Pflanzen

Kunststofftöpfe werden mit expandiertem Vermiculit (Dichte: 0,135 g/cm$^3$, Wasserabsorptionsvermögen: 0,565 l/l) gefüllt. Nach dem Sättigen des nicht adsorptiven Vermiculits mit einer wässrigen Wirkstoffemulsion in deinonisiertem Wasser, die die Wirkstoffe in einer Konzentration von 70,8 ppm enthält, werden Samen der folgenden Pflanzen auf die Oberfläche gesät. Nasturtium offcinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während der Keimphase von 4 bis 6 Tagen werden die Töpfe zur Erhöhung der örtlichen Luftfeuchtigkeit mit lichtdurchlässigem Material abgedeckt und mit deinoisiertem Wasser begossen. Nach dem 5. Tag wird dem Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen bewertet.

In diesem Versuch zeigen die Verbindungen der Tabellen 1 bis 6 eine sehr starke Herbizidwirkung.

Beispiel B4 Herbizidwirkung für Wasserreis (paddy)

Die Wasserunkräuter Echinochloa crus galli und Monocharia vaginalis werden in Plastikbechern (60 cm$^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe. Die dabei verwendete Dosis entspricht einer Wirkstoffmenge von 4 kg AS pro Hektar (Spritzbrühmenge = 550 l/ha). Die Pflanzenbecher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation nach der gleichen Notenskala wie im preemergenten Versuch statt. Die Verbindungen der Tabellen 1 bis 6 schädigen dabei die Unkräuter, nicht aber den Reis.

Testresultate: postemergente Prüfung

Aufwandmenge: 4 kg Aktivsubstanz pro Hektar

| Verb. Nr. | Echinochloa | Monochoria |
|---|---|---|
| 1.016 | 1 | 1 |
| 2.034 | 1 | 1 |
| 3.03 | 1 | 1 |

**Patentansprüche**

1. Imidazol-Derivate der Formel I

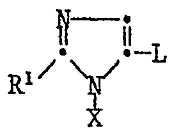 (I),

stereochemisch isomere Formen davon, sowie deren Salze, worin X einen Rest der Formel

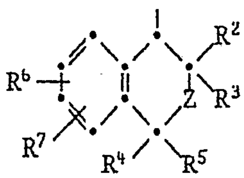 oder 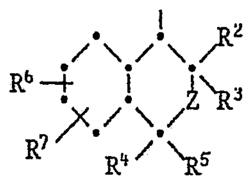

$R^1$ Wasserstoff oder -SH, und
L Cyano, -COOH, -COOR$^8$. -COSR$^8$, -CONR$^9$R$^{10}$, -CO-R$^{11}$, -$CH_2$-O-R$^{12}$ oder eine Gruppe

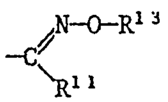 oder 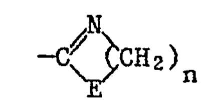 bedeuten, wobei

bedeuten, wobei E für Sauerstoff, Schwefel, -N($C_1$-$C_4$Alkyl)- oder -NH- ,
n für die Zahl zwei oder drei,
Z für Sauerstoff, Schwefel, -SO-, -$SO_2$- oder -NR$^{14}$-,
$R^2$ und $R^3$ unabhängig voneinander für Wasserstoff, $C_1$-$C_4$-Alkyl, $C_2$-$C_4$-Alkenyl oder $C_2$-$C_4$-Alkinyl oder gemeinsam für eine spirocyclisch verknüpfte $C_2$-$C_6$-Alkylenkette,
$R^4$ und $R^5$ unabhängig voneinander für Wasserstoff oder $C_1$-$C_4$-Alkyl oder gemeinsam für eine Oxofunktion,
$R^6$ für Wasserstoff, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio oder Nitro,
$R^7$ für Wasserstoff, $C_1$-$C_4$-Alkoxy, Halogen oder $C_1$-$C_4$-Alkyl,
$R^8$ für $C_1$-$C_6$-Alkyl, $C_3$-$C_7$-Cycloalkyl, $C_2$-$C_6$-Alkoxyalkyl, Benzyl, -CR$^{15}$R$^{16}$-$C_2$-$C_4$-Alkenyl oder -CR$^{15}$R$^{16}$-$C_2$-$C_4$-Alkinyl,
$R^9$ für Wasserstoff, $C_1$-$C_4$-Alkyl, $C_1$-$C_4$-Alkoxy, Phenyl, Benzyl, -CR$^{15}$R$^{16}$-$C_2$-$C_4$-Alkenyl oder -CR$^{15}$R$^{16}$-$C_2$-$C_4$-Alkinyl,
$R^{10}$ für Wasserstoff oder $C_1$-$C_4$-Alkyl, oder
$R^9$ und $R^{10}$ zusammen mit dem sie tragenden Stickstoffatom für einen Pyrrolidin-, Piperidin- oder Morpholinrest,
$R^{11}$ für $C_1$-$C_6$-Alkyl, $C_3$-$C_7$-Cycloalkyl, $C_1$-$C_4$-Halogenalkyl oder gegebenenfalls durch $C_1$-$C_4$-Alkyl, $C_1$-$C_4$-Alkoxy, Nitro oder Halogen substituiertes Phenyl,
$R^{12}$ für Wasserstoff, $C_1$-$C_6$-Alkyl, gegebenenfalls durch $C_1$-$C_4$-Alkyl, $C_1$-$C_4$-Alkoxy, Halogen oder Nitro substituiertes Phenyl, $C_1$-$C_6$-Alkylcarbonyl, $C_1$-$C_4$-Halogenalkylcarbonyl oder gegebenenfalls durch $C_1$-$C_4$-Alkyl, $C_1$-$C_4$-Alkoxy, Halogen oder Nitro substituiertes Benzoyl,
$R^{13}$ für $C_1$-$C_4$-Alkyl, -CR$^{15}$R$^{16}$-$C_2$-$C_4$-Alkenyl, Benzyl oder -CR$^{15}$R$^{16}$-$C_2$-$C_4$-Chloralkenyl,
$R^{14}$ für Wasserstoff, $C_1$-$C_4$-Alkyl, $C_1$-$C_4$-Alkylcarbonyl, $C_1$-$C_4$-Alkoxycarbonyl, Benzyloxycarbonyl, Benzoyl oder Benzyl,
und
$R^{15}$ und $R^{16}$ unabhängig voneinander für Wasserstoff oder $C_1$-$C_4$-Alkyl stehen,

2. Verbindungen gemäss Anspruch 1, dadurch gekennzeichnet, dass $R^1$ für Wasserstoff steht.

3. Verbindungen gemäss Anspruch 1, dadurch gekennzeichnet, dass L für -COOR$^8$, -CO-NR$^9$R$^{10}$ oder -CO-R$^{11}$ steht.

4. Verbindungen gemäss Anspruch 3, dadurch gekennzeichnet, dass L für $C_1$-$C_4$-Alkoxycarbonyl steht.

5. Verbindungen gemäss Anspruch 3, dadurch gekennzeichnet, dass L für -CO-NR$^9$R$^{10}$steht, worin $R^9$ und

$R^{10}$ unabhängig voneinander Wasserstoff oder $C_1$-$C_4$-Alkyl bedeuten.

6. Verbindungen gemäss Anspruch 3, dadurch gekennzeichnet, dass L für $C_1$-$C_4$-Alkylcarbonyl steht.

7. Verbindungen gemäss Anspruch 1, dadurch gekennzeichnet, dass X für den Rest

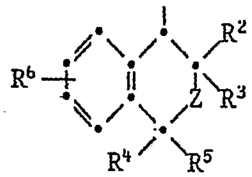

steht, worin

steht, worin $R^2$ und $R^3$ unabhängig voneinander Wasserstoff oder $C_1$-$C_4$-Alkyl, $R^4$ und $R^5$ WAsserstoff oder gemeinsam für eine Oxofunktion, $R^6$ Wasserstoff, $C_1$-$C_4$-Alkyl, $C_1$-$C_4$-Alkoxy oder Halogen und Z Sauerstoff, -NH-, -$NCH_3$-oder Schwefel bedeuten.

8. Verbindungen gemäss Anspruch 1, dadurch gekennzeichnet, dass X für den Rest

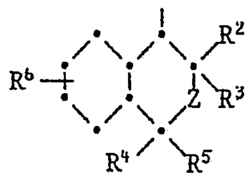

steht, worin

steht, worin die Ringverknüpfung in trans-Stellung vorliegt, $R^2$ und $R^3$ unabhangig voneinander Wasserstoff oder $C_1$-$C_4$-Alkyl, $R^4$ und $R^5$ Wasserstoff, $R^6$ Wasserstoff, $C_1$-$C_4$-Alkyl oder Halogen und Z Sauerstoff, -NH-, -$NCH_3$-oder Schwefel bedeuten.

9. Verbindungen gemäss Anspruch 7, dadurch gekennzeichnet, dass $R^1$ für Wasserstoff und L für $C_1$-$C_4$-ALkoxycarbonyl, $C_1$-$C_4$-Alkylcarbonyl, Carbamoyl, Methylcarbamoyl oder Dimethylcarbamoyl stehen.

10. Verbindungen gemäss Anspruch 8, dadurch gekennzeichnet, dass $R^1$ für Wasserstoff und L für $C_1$-$C_4$-Alkoxycarbonyl, $C_1$-$C_4$-Alkylcarbonyl, Carbamoyl, Methylcarbamoyl oder Dimethylcarbamoyl stehen.

11. 1-(3-Methyl-isochroman-4-yl)-5-imidazolcarbonsäure-methylester gemäss Anspruch 1.

12. 1-(3,3-Dimethyl-isochroman-4-yl)-5-imidazolcarbonsäure-methylester gemäss Anspruch 1.

13. 1-(3-Methyl-isothiochroman-4-yl)-5-imidazolcarbonsäure-methylester gemäss Anspruch 1.

14. 1-(3,3-Dimethyl-isothiochroman-4-yl)-5-imidazolcarbonsäure-methylester gemäss Anspruch 1.

15. 1-(3-Methyl-1,2,3,4-tetrahydroisochinolin-4-yl)-5-imidazolcarbonsäure-methylester gemäss Anspruch 1.

16. 1-(2,3-Dimethyl-1,2,3,4-tetrahydroisochinolin-4-yl)-5-imidazolcarbonsäure-methylester gemäss Anspruch 1.

17. 1-(3,3-Dimethyl-1,2,3,4-tetrahydroisochinolin-4-yl)-5-imidazolcarbonsäure-methylester gemäss Anspruch 1.

18. 1-(3,3-Dimethyl-1-oxo-isochroman-4-yl)-5-imidazolcarbonsäure-methylester gemäss Anspruch 1.

19. Verfahren zur Herstellung der Verbindungen der Formel I, worin $R^1$ für Wasserstoff steht, dadurch gekennzeichnet, dass man ein Amin der Formel II

$X-NH_2$ (II)

worin X die unter Formel I im Anspruch 1 gegebene Bedeutung hat, mit einem N-Cyan-Imidoameisensäureester der Formel III

$R-O-CH=N-CN$ (III)

worin R für $C_1$-$C_4$-Alkyl steht, kondensiert, das entstehende N-Cyanoformamidin der Formel IV

$X-NH-CH=N-CN$ (IV)

worin X die unter Formel I im Anspruch 1 gegebene Bedeutung hat, mit einem Halogenessigsäureester der Formel V

$Hal-CH_2-L$ (V)

worin Hal für Chlor oder Brom und L für $C_1$-$C_6$-Alkoxycarbonyl stehen, in Gegenwart einer Base alkyliert, das entstehende Zwischenprodukt der Formel VI

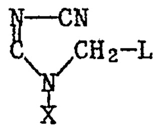

(VI)

worin X die unter Formel I im Anspruch 1 gegebene Bedeutung hat und L für $C_1$-$C_6$-Alkoxycarbonyl steht, in Gegenwart einer Base zum 4-Aminoimidazol der Formel VII

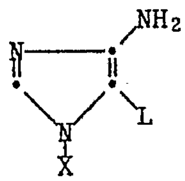

(VII)

worin X die unter Formel I im Anspruch 1 gegebene Bedeutung hat und L für $C_1$-$C_6$-Alkoxycarbonyl steht, cyclisiert und diese Verbindung unter Diazotierung und Stickstoffabspaltung in das Produkt der Formel I, worin L für $C_1$-$C_6$-Alkoxycarbonyl und $R^1$ für Wasserstoff stehen, überführt und gewünschtenfalls die Gruppe L in 5-Stellung gemäss den übrigen Bedeutungen der Definition unter Formel I im Anspruch 1 derivatisiert.

20. Verfahren zur Herstellung der Verbindungen der Formel I, dadurch gekennzeichnet, dass man ein Amin der Formel II

$X$-$NH_2$ (II)

worin X die unter Formel I im Anspruch 1 gegebene Bedeutung hat, mit einem Halogenessigsäureester der Formel V

Hal-$CH_2$-L (V)

worin Hal für Chlor oder Brom und L für $C_1$-$C_6$-Alkoxycarbonyl stehen, in Gegenwart einer Base alkyliert, den entstehenden Glycinester der Formel VIII

X-NH-$CH_2$-L (VIII)

worin X die unter Formel I im Anspruch 1 gegebene Bedeutung hat und L für $C_1$-$C_6$-Alkoxycarbonyl steht, in Gegenwart von Essigsäureanhydrid mit Ameisensäure formyliert, das entstehende Zwischenprodukt der Formel IX

HCO- -$CH_2$-l (IX)

worin X die unter Formel I im Anspruch 1 gegebene Bedeutung hat und L für $C_1$-$C_6$-Alkoxycarbonyl steht, in Gegenwart einer Alkalibase mit einem Ameisensäure-$C_1$-$C_4$-alkylester umsetzt, das entstehende Zwischenprodukt der Formel X

HCO- – =CH-O-M (X)

worin X die unter Formel I gegebene Bedeutung hat und L für $C_1$-$C_6$-Alkoxycarbonyl und M für ein Alkalikation steht, mit Thiocyansäure zum Produkt der Formel I, worin $R^1$ für -SH und L für $C_1$-$C_6$-Alkoxycarbonyl stehen, cyclisiert und diese Verbindung gewünschtenfalls in der 2-Stellung desulfuriert und gewünschtenfalls die Gruppe L in 5-Stellung gemäss den übrigen Bedeutungen der Definition unter Formel I im Anspruch 1 derivatisiert.

21. Herbizides Mittel, dadurch gekennzeichnet, dass es einer wirksamen Menge einer Verbindung der Formel I einen oder mehrere Träger- und/oder Zuschlagstoffe enthält.

22. Verfahren zur Bekämpfung von unerwünschtem Pflanzenwuchs, dadurch gekennzeichnet, dass man die Pflanzen oder deren Standort mit einer herbizid wirksamen Menge eines Wirkstoffs der Formel I behandelt.

23. Verfahren zur selektiven Bekämpfung von Unkräutern in Nutzpflanzenkulturen, dadurch gekennzeichnet, dass man eine für die Unkräuter herbizid wirkende Menge eines Wirkstoffs der Formel I auf die Nutzpflanzenkulturen appliziert.

24. Verfahren gemäss Anspruch 23, dadurch gekennzeichnet, dass es sich bei den Nutzpflanzenkulturen um

Mais-, Reis- oder Getreidekulturen handelt.

25. Verfahren gemäss Anspruch 23, dadurch gekennzeichnet, dass es sich bei der Nutzpflanzenkultur um Reis handelt.

26. Amine der Formel

$H_2N-X$ (II)

worin X die unter Formel I im Anspruch 1 gegebene Bedeutung hat, unter Ausnahme der Verbindungen 4-Amino-2-methyl-1,2,3,4-tetrahydroisochinolin, 4-Amino-2-äthyl-1,2,3,4-tetrahydroisochinolin, 4-Amino-2-benzyl-1,2,3,4-tetrahydroisochinolin und 4-Amino-7-methoxy-1,2,3,4-tetrahydroisochinolin.

27. 4-Amino-imidazole der Formel VII

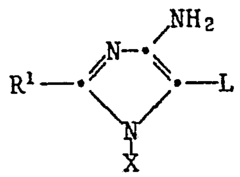

(VII)

worin $R^1$, X und L die unter Formel I im Anspruch 1 gegebene Bedeutungen haben.

28. Oxime der Formel XI

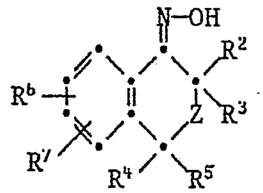

(XI)

worin $R^2$, $R^3$, $R^4$, $R^5$, $R^6$, $R^7$ und Z die unter Formel I im Anspruch 1 gegebenen Bedeutungen haben, mit der Massgabe, dass $R^2$ und $R^3$ nicht gleichzeitig für Wasserstoff stehen.

29. Oxime der Formel XII

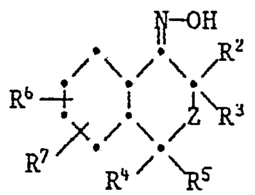

(XII)

worin $R^2$, $R^3$, $R^4$, $R^5$, $R^6$, $R^7$ und Z die unter Formel I im Anspruch 1 gegebenen Bedeutungen haben.